(19) Europäisches Patentamt

European Patent Office

Office européen des brevets

(11) **EP 1 492 019 A1**

(12) **EUROPEAN PATENT APPLICATION**
published in accordance with Art. 158(3) EPC

(43) Date of publication:
29.12.2004 Bulletin 2004/53

(51) Int Cl.[7]: **G06F 17/30**, A61K 45/00,
A61P 43/00

(21) Application number: 03708546.1

(22) Date of filing: 11.03.2003

(86) International application number:
PCT/JP2003/002847

(87) International publication number:
WO 2003/077159 (18.09.2003 Gazette 2003/38)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 11.03.2002 JP 2002064994

(71) Applicant: Institute of Medicinal Molecular
Design, Inc.
Tokyo 113-0033 (JP)

(72) Inventors:
• TOMIOKA, Nobuo
Bunkyo-ku, Tokyo 113-0 (JP)
• ITAI, Akiko
Bunkyo-ku, Tokyo 113-0 (JP)

(74) Representative:
Sternagel, Fleischer, Godemeyer & Partner
Patentanwälte,
An den Gärten 7
51491 Overath (DE)

(54) **METHOD OF FORMING MOLECULE FUNCTION NETWORK**

(57) A method of generating a molecule-function network comprising a step of a connect search using a database which stores information on biomolecules hierarchized by one or more items including items selected from a group consisting of modification state, active or inactive state, complexation state, and structural change. By using the biomolecule-linkage database of the present invention, which is a collection of information on biomolecule pairs including bio-events, it is possible to generate a molecule-function network with a necessary range which is a functional or biosynthetic linkage between molecules and to predict bio-events to which an arbitrary biomolecule is related directly or indirectly.

Fig. 1

**Description**

Technical Field

The present invention relates to a method of creating and using a biomolecule database including bio-event information.

Background Art

In an organism, various molecules such as amino acids, nucleic acids, lipids, carbohydrates and general small molecules as well as biomolecules such as DNA, RNA, proteins and polysaccharides exist, and each bears its function. Characteristics of a biological system are not only that it is constituted of various biomolecules, but also that all phenomena in an organism such as an expression of a function occur through a specific binding between biomolecules. In such specific binding, a covalent bond is not formed, instead, a stable complex is formed by intermolecular force. Therefore, a biomolecule exists in equilibrium between an isolated state and a complex state, and between certain biomolecules, a stability of a complex state is greater and the equilibrium is remarkably biased to a complex side. As a result, in the existence of many other molecules, a molecule can distinguish and bind to a specific partner practically even in a fairly diluted concentration. In enzyme reactions, a substrate is released as a reaction product after receiving a specific chemical conversion in a complex state with an enzyme, and in signal transduction, an extracellular signal is transmitted into a cell through a structural change of a target biomolecule which occurs upon binding of a mediator molecule to the target biomolecule.

Recently, a progress of genome study is remarkable, genome sequences of various species including human have been elucidated, and genome-wide systematic studies are underway for genes and sequences of proteins that are the products of genes, expression of proteins in each organ, protein-protein interactions and others. Most of the results of these studies are open to public as databases, and are available for use throughout the world. Elucidation is progressing little by little regarding functions of genes and proteins, prediction of a gene that causes or is a background of a disease, and a relation with gene polymorphism. Consequently, medical treatment and drug development based on genetic information are more and more anticipated.

Most biological functions such as energy metabolism, substance conversion and signal transduction are born by molecules other than a nucleic acid, whereas the genetic information is born by the nucleic acid. A protein is different from molecules of other categories in a point where it is directly produced based on a design chart called gene, and there are many kinds of proteins. Enzymes, target biomolecules of an intrinsic physiologically-active compound, target biomolecules (modified with sugar in many cases) of an intrinsic physiologically-active protein are all proteins. Set a primary cause of a disease aside, it is considered that many diseases and symptoms are results of abnormalities of the amount or balance, or quality (function) in some cases, of a protein or a small molecule. Most of the existing drugs are compounds that act to a protein as a target and control its functions. Different from a protein, a steric structure of a nucleic acid is not suitable for acting specifically as a target of a small molecular drug. Targets of antibiotics and antibacterial agents as well as agrochemicals such as insecticides and antimycotic agents are proteins.

Therefore, it is necessary to clarify functions of proteins and small molecules in an organism and specific relations among those molecules, in order to carry out medical treatment or drug development based on the genetic information. Since different enzymes play their parts one after another in biosynthesis of a necessary molecule and since different molecules bind together in turn in signal transduction, these molecules have direct or indirect, functional or biosynthetic, mutual linkage, hence information on the linkage (molecule-function network) is important. Many molecules such as mediators and hormones which directly involve in occurrences of various clinical symptoms, physiological phenomena, and biological responses have been discovered with the studies so far, and it is inevitable to correlate those molecules with a molecule-function network for performing an appropriate treatment. In a strategy for drug development, it is necessary to consider a molecule-function network including target molecules, in order to select an appropriate target molecule for drug development while considering a risk of side effects.

As databases related to proteins, SwissProt (the Swiss Institute of Bioinformatics (SIB), European Bioinformatics Institute (EBI)) and PIR (National Biomedical Research foundation (NBRF)) are known, and both contain annotation information on species, function, functional mechanism, discoverer, literature and others as well as sequence information.

Among molecule-network databases focusing on the linkage of molecules, KEGG (Kanehisa et al., Kyoto University), Biochemical Pathways (Boehringer Mannheim), WIT (Russian Academy of Sciences), Biofrontier (Kureha Chemical Industry), Protein Pathway (AxCell), bioSCOUT (LION), EcoCyc (DoubleTwist), and UM-BBD (Minnesota Univ.) are known as databases about metabolic pathways.

The PATHWAY database of KEGG contains metabolic pathways and signal transduction pathways, wherein the former treats metabolic pathways of general small molecules involved in substance metabolism and energy metabolism, and the latter treats proteins of signal transduction system. In both, pre-defined molecule networks are provided as static Gif files. In the former, information on enzymes and ligands is imported from separate text-style molecule databases, LIGAND (Kanehisa et al., Kyoto Univ.) and ENZYME (IUPAC-IUBMB). Information on enzymes involved in syntheses of physiologically active peptides and information on target biomolecules are not included.

EcoCyc is a database of substance metabolism in Escherichia coli, and it displays a pathway as a diagram based on data about individual enzyme reactions and data about known pathways (represented as a collection of enzyme reactions belonging to the pathway). Search by a character string or an abbreviated symbol for a molecule name or a pathway name is provided as a search function of EcoCyc, however, it is not possible to search a new pathway by specifying an arbitrary molecule.

Those concerning signal transduction, CSNDB (National Institute of Health Sciences, Japan), SPAD (Kuhara et al., Kyushu Univ.), Gene Net (Institute of Cytology & Genetics Novosibirsk, Russia), and GeNet (Maria G. Samsonova) are known. As databases of protein-protein interaction, DIP (UCLA), PathCalling (CuraGen), and ProNet (Myriad) are known. As databases of expressions of gene or protein, BodyMap (Univ. of Tokyo and Osaka Univ.), SWISS-2DPAGE (Swiss Institute of Bioinformatics), Human and mouse 2D PAGE database (Danish Centre for Human Genome Research), HEART-2DPAGE (GermanHeart), PDD Protein Disease Databases (NIMH-NCI), Washington University Inner Ear Protein Database (Washington Univ.), PMMA-2DPAGE (Purkyne Military Medical Academy), Mito-Pick (CEA, France), Molecular Anatomy Laboratory (Indiana University), and Human Colon Carcinoma Protein Database (Ludwig Institute for Cancer Research) are known. As examples of molecule network for biological response simulation, E-Cell (Tomita et al., Keio Univ.), e E.coli (B. Palsson), Cell (D. Lauffenburger, MIT), Virtual Cell (L. Leow, Connecticut Univ.), and Virtual Patient (Entelos, Inc.) are known.

Concerning relations between biomolecules and functions, SwissProt collects broad information on protein, and COPE (University of Munich) provides information on functions of cytokines in a text format. ARIS (Japan Information Processing Service Co. Ltd.) records literature information on side effects and interactions of drugs and on toxication by agrochemicals and chemicals gathered from approximately 400 domestic journals and 20 foreign journals mostly on medical and pharmacological fields. However, a database for physiological actions and responses above cellular level of biomolecules has not been available so far. Concerning genes and diseases, OMIM (NIH) collects information on genetic diseases and amino acid mutations of proteins. The data is described in a text format and can be searched by a keyword.

Problems of the existing databases focusing on linkages between molecules are as follows. Molecule-network databases have been prepared for systems in which molecules included and linkages between the molecules are known. Since it is possible to arrange molecules beforehand considering the relation between the molecules, static representation such as Gif has been sufficient. However, with such a method, it is difficult to add new molecules and linkages between the molecules. There exist more than 100,000 molecules including molecules that will be revealed in the future (the number of molecules that KEGG treats is about 10,000 including drug molecules), and when the linkages between those molecules will be elucidated in the future research, it is expected that the complexity of the molecule network will increase exponentially. We need a new method that is well adapted to additions of new molecules, and can generate a partial molecule network containing necessary information while retaining information on huge number of molecules and relations between the molecules.

As of Sept. 7, 2001, KEGG stores linkages between molecules as information on pairs of two molecules, and it is possible to search for a pathway that links arbitrary two molecules in metabolic pathways using that information. However, pathway search problem like this has difficulty that the longer the pathway linking the two molecules, the exponentially more the computation time.

On the other hand, there is no limit to additions of molecule data in a text database. However, it is difficult to generate a molecule network representing linkages of many molecules by repeating searches one after another for functionally or biosynthetically related molecules from a data of each molecule. It is necessary to develop methods of storing and searching data so that linkages for necessary molecules are obtained dynamically and automatically at the time of the search. Furthermore, in order to understand diseases and pathological states at molecular level, we need a new invention to describe relations between biomolecule / molecule network and biological responses / physiological actions.

Disclosure of Invention

An object of the present invention is to provide schemes and methods to understand various biological responses and phenomena in the light of the functions of biomolecules and relations between those molecules, and to be more specific, to provide databases and search methods that can link information on biomolecules to biological responses. Furthermore, one of the other objects of the present invention is to provide a method of extracting rapidly and efficiently, from huge amount of information, only signal transduction pathways and biosynthetic pathways related to an arbitrary biological response or biomolecule, and predicting a promising drug target and a risk of side effects.

As a result of zealous endeavor to solve the aforementioned object, the inventors found that the aforementioned object can be solved by covering linkages between biomolecules by accumulating information on a pair of direct-binding biomolecules which is treated as a part, by attaching information on bio-events comprising physiological actions, biological responses, clinical symptoms and others to the pair between a key molecule involved directly in the expression of a biological response and its target biomolecule, and by generating a molecule-function network by searching linkages that include designated one or more arbitrary biomolecules or bio-events automatically and consecutively. They filed a patent application for the aforementioned inventions (the specification of PCT/JP01/07830).

That is, a method of generating a molecule-function network by using a biomolecule-linkage database that accumulates information on direct-binding biomolecule pairs is described in the PCT/JP01/07830 specification. In preferred embodiments of this invention, there are provided the aforementioned method which generates a molecule-function network related with information on bio-events by using biomolecule-linkage database comprising information on bio-events; the aforementioned method which uses a biomolecule-information database comprising information on biomolecules themselves; and the aforementioned method which generates a molecule-function network including drug molecules related with information on bio-events. Furthermore, the invention also provides a method of predicting bio-events directly or indirectly related to an arbitrary biomolecule or a drug molecule by using a biomolecule-linkage database which accumulates information on bio-events related to a direct-binding biomolecule. Moreover, the invention provides a method of analyzing information on polymorphism or expression of genes using a molecule-function network, by generating a database which links a molecule ID of a biomolecule with a name, an ID, or an abbreviated name of a gene when the biomolecule is a protein coded by the gene in an external database or a literature.

In more preferred embodiments of the above-mentioned invention, there are provided the aforementioned method characterized by hierarchizing the molecule-function network based on the belonging subnet and inclusion relationships among subnets wherein biomolecule pairs grouped based on the linkage on the network are treated as a subnet; the aforementioned method characterized by hierarchical storage of information on biomolecule pairs based on belonging pathway name, belonging subnet name and others; the aforementioned method characterized by hierarchical storage of information on biomolecules themselves based on expression patterns from genes and expression patterns on cell surface and others; and the aforementioned method characterized by hierarchical storage of information on bio-events based on classification by the superordinate concept of said event and/or based on the relation with pathological events. Furthermore, there are also provided by the above-mentioned invention, the aforementioned method characterized by storage of information on relationship and dependence among items stored at upper hierarchy comprising upper hierarchy of biomolecule pairs, upper hierarchy of biomolecules themselves and upper hierarchy of bio-events; the aforementioned method characterized by facilitating generation of a molecule-function network using hierarchical information stored in a biomolecule information database or a biomolecule-linkage database; and the aforementioned method characterized by controlling the details in representation of a molecule-function network using hierarchical information stored in a biomolecule information database or biomolecule-linkage database.

As a result of zealous endeavor, the inventors succeeded in providing the following methods and databases.

1. A molecule-function network generated by carrying out a connect search using a biomolecule-linkage database while filtering biomolecule pairs by one or more combinations of data items such as a relation code, a relation-function code, a reliability code, an acting organ, directionality of a biomolecule pair; or a method of generating said molecule-function network.

2. A method of screening the molecule-function network generated by a connect search using a biomolecule-linkage database by scoring with one or more combinations of data items such as a relation code, a relation-function code, a reliability code, an acting organ, directionality of a biomolecule pair.

3. A method of highlighting an item corresponding to a data item included in any one of the biomolecule information database, biomolecule-linkage database, drug molecule information database, drug molecule-linkage database, and pathology-linkage database, when said item exists in the information being viewed on a terminal.

4. A method of highlighting an item corresponding to any data item in the molecule-function network generated by a connect search, when said item exists in the information being viewed on a terminal.

5. A method of displaying database information containing an item corresponding to a data item included in any one of the biomolecule information database, biomolecule-linkage database, drug molecule information database, drug molecule-linkage database, and pathology-linkage database, when said item exists in the information being viewed on a terminal.

6. A method of highlighting an item in a molecule-function network corresponding to any data item in the molecule-function network generated by a connect search, when said item exists in the information being viewed on a terminal.

7. A method of generating a molecule-function network characterized in that the range of the generated molecule-function network is limited by the number of paths.

8. A method of generating a molecule-function network connecting three or more molecules by repeating connect searches between two molecules and by combining the result of said searches.

9. A method of generating a molecule-function network characterized in that a connect search is carried out while filtering biomolecules based on the information on the originating organ and/or the existing organ of the biomolecules.

10. A method of generating a molecule-function network characterized in that a connect search is carried out while filtering biomolecules based on the information on the amount of expression of biomolecules and/or the amount of transcription of genes.

11. A method of generating a molecule-function network characterized in that the information on two or more species is used in combination.

12. A method of analyzing the experimental data of a knockout animal or a transgenic animal using the aforementioned method of 11.

13. A method of predicting an influence of the knocked-out gene in a knockout animal or an influence of the introduced gene in a transgenic animal using the aforementioned method of 11.

Brief Description of the Drawings

Figure 1 shows a basic concept of the method of the present invention.
Figure 2 shows a result of a connect search containing paths with different path lengths.
Figure 3 shows a result of a connect search designating one search point.
Figure 4 shows a result of a connect search designating three or more search points.
Figure 5 shows a concept when a drug molecule-linkage database is used in the method of the present invention.
Figure 6 shows a concept when a genetic information database is used in the method of the present invention.
Figure 7 shows a method of registering and using information on a complex to the biomolecule information database in Example 1.
Figure 8 shows a method of correlating a complex state and an isolated state of a biomolecule in Example 1.
Figure 9 shows a method of describing modification states by hierarchization of the biomolecule data in Example 2.
Figure 10 shows a method of merging different modification states of a biomolecule in Example 3.
Figure 11 shows a concept of the relationship of data items in the pathology-linkage database taking diabetes as an example.
Figure 12 shows relationships between subnets in the molecule-function network obtained by the search method of Example 5 and key molecules in the pathology-linkage database
Figure 13 shows a method of examining a mechanism of a disease from disease names and pathological events in Example 6 using a molecule-function network.
Figure 14 shows a method of searching a subnet from a disease name via a key molecule in Example 7.
Figure 15 shows a method of examining a function and a role of a biomolecule in an organism in Example 8.
Figure 16 shows the search result of Example 9 using a pathology-linkage database.
Figure 17 shows a method of examining a mechanism of action of a drug molecule based on target biomolecule information in the drug molecule information database in Example 10.
Figure 18 shows a method of examining details of a mechanism of action of a drug molecule through biomolecules, bio-events, and subnets in Example 11.

Detailed Description of the Preferred Embodiments

It is useful to refer to the disclosures of the specification of PCT/JP01/07830 for understanding the present invention. All of the disclosures of the aforementioned specification of PCT/JP01/07830 are herein incorporated by reference.
Meanings or definitions of the terms in the present description are as follows.
"Organism" is a concept including the whole or a part of an organism, for example, organelle, cell, tissue, organ, individual, a group of individuals, as well as parasite.
"Bio-event" is a concept including all phenomena, responses, reactions, and symptoms appearing endogenously or exogenously in an organism. Transcription, cell migration, cell adhesion, cell division, neural excitation, vasoconstriction, increase of blood pressure, decrease of blood glucose level, fever, convulsion, infection by a parasite such as a heterogeneous organism and a virus can be pointed out as specific examples. Furthermore, physical stimulations such as light and heat from outside of an organism and responses of an organism to them may be included in the concept of bio-event.
"Pathological event" is a concept that can be included in the "bio-event," and means a condition where a "bio-event" exceeds a certain threshold quantitatively or qualitatively, and can be judged as a disease or a pathological state. For example, high blood pressure or hypertension can be pointed out as "pathological events" as a consequence of an extraordinary increase in the "bio-event" of blood pressure increase, and hyperglycemia or diabetes can be pointed out as "pathological events" wherein blood sugar is not controlled within a normal range. There are pathological events that are related to multiple kinds of bio-events, as well as the aforementioned examples that are related to a single bio-event. Furthermore, biological response, symptom, syndrome, abnormality in clinical marker value, complication observed in a disease are also included in the concept of the pathological event. "Biomolecule" indicates an organic molecule of various structures or a molecular complex existing in an organism, such as a nucleic acid, a protein, a lipid, a carbohydrate, a general small molecule, and may contain metal ion, water, and a proton as well.

"Key molecule" mainly indicates a molecule such as a mediator, a hormone, a neurotransmitter and an autacoid, among biomolecules. In most cases, a specific target biomolecule exists in an organism to a key molecule, and it is known that a direct binding of the key molecule to the target molecule acts as a trigger of the aforementioned "bio-event." Although key molecules are generated and exerting actions in an organism, a bio-event is generally expressed corresponding to the given amount even when they are given from outside of an organism. Adrenalin, angiotensin II, insulin, estrogen and others can be pointed out as specific examples.

Furthermore, even if the target biomolecule has not been confirmed or the direct binding to the target biomolecule has not been confirmed for a biomolecule, the biomolecule may be treated as a "key molecule" when it is known that a quantitative or a qualitative change of said biomolecule leads to an exaltation or increase, or suppression or decrease of a bio-event, or gives a fluctuation to a molecule-function network or a subnet.

"Target biomolecule" means a specific biomolecule that can accept a biomolecule such as a mediator, a hormone, a neurotransmitter, and an autacoid (most of which are the key molecules) or a drug molecule. In most cases, these biomolecules or drug molecules express a specific bio-event by binding directly to the target molecule. Furthermore, even in the case where the direct binding has not been confirmed, a biomolecule may be regarded as a target biomolecule if a quantitative or a qualitative change of the biomolecule is observed when a certain biomolecule or a drug molecule is given to an organism.

"Up-or-down information of a bio-event" is the information on exaltation /increase or suppression / decrease in response to a quantitative or qualitative change of a key molecule or a target biomolecule. It includes a case where the bio-event occurs only after the amount of the key molecule exceeds a certain threshold.

"Molecule ID" is given for the purpose of identification or designation of a molecule instead of the molecule name, and needs to correspond to each molecule uniquely. An abbreviated symbol of a molecule name or an alphanumeric character string irrelevant to a molecule name may be acceptable, however, it is desirable to use a short character string. When there is a molecule ID that is already used globally, it is desirable to use it. It is possible to give multiple molecule IDs assigned by different methods to one molecule and to hierarchize them by structural group or function or choose between them as necessary.

"Direct binding" means formation of a stable complex by an intermolecular force not by a covalent bond, or means possibility of complex formation. A covalent bond is formed in a rare case, and such a case is included in this concept. Even in the case where a stable complex is not formed as in the relation between an enzyme and a product in an enzyme reaction, if two or more molecules are known or expected to have a specific relation in an organism, the relation between said molecules may be regarded as direct binding. For a combination between a transcription factor and a protein whose expression is induced or suppressed by said transcription factor, it may be regarded as a direct binding if the combination has been established. The concept of "direct binding" is often called "interaction", however, interaction includes broader meanings.

"Biomolecule pair" means a pair of biomolecules capable of direct binding or presumed to form direct binding in an organism. Estradiol and estrogen receptor, angiotensin converting enzyme and angiotensin I can be pointed out as specific examples. In a case of a molecule pair of an enzyme and a product in an enzyme reaction, the complex is not very stable but can be included in biomolecule pairs. The relation between a transcription factor and a protein whose expression is induced or suppressed by said transcription factor can be also included in biomolecule pairs. Furthermore, a molecules pair whose mutual role is not clear, like two protein molecules judged to have interaction by the two-hybrid experimental technique, may be included. For physical or chemical stimulations from outside of an organism such as light, sound, temperature change, magnetic field, gravity, pressure and vibration, these stimulations may be treated as virtual biomolecules, and a biomolecule pair to a corresponding target biomolecule may be defined.

"To correlate" means indicating or recording a direct or an indirect relation between any two data items among biomolecules, subnets, bio-events, pathological events, drug molecules, genes, and data items related to diseases. "Relation information" is the information recorded by "correlating".

"Structure code" is a classification code representing structural features whether a biomolecule is DNA, RNA, a protein, a peptide, or a general small molecule and others. Regarding a protein, structural features such as 7-transmembrane type, 1-transmembrane type, beta-barrel may be further described hierarchically.

"Function code" is a classification code representing a function of a biomolecule at molecular level, for example, in the case of a biomolecule wherein the "structure code" is "protein", it represents a classification of membrane receptor /nuclear receptor / transporter / mediator / hydrolase / kinase / phosphorylase and others, and in the case of a biomolecule wherein the "structure code" is "small molecule", it represents a classification of substrate / product / precursor / active peptide / metabolite and others. Furthermore, regarding a protein, subordinate classification of functions may be described hierarchically, for example, serine-threonine kinase, tyrosine kinase, MAP kinase and others, for a kinase.

"Relation code" is a classification code representing a relation between two molecules constituting a biomolecule pair. It may be categorized, for example, 10 for an agonist and a receptor, 21 for an enzyme and a substrate, 22 for a substrate and a product. If a mutual role of two molecules is not clear, as in the case of two protein molecules considered to have an interaction by the two-hybrid experimental technique, it is desirable to use a code representing such a situation. Regarding the relation between a transcription factor and a protein whose expression is induced or suppressed by said transcription factor, it is desirable to use a distinct code indicating such a relation.

"Relation-function code" is a classification code representing a phenomenon or a change accompanied by a direct binding of two molecules constituting a biomolecule pair; for example, a classification such as hydrolysis, phosphorylation, dephosphorylation, activation, inactivation may be used.

"Reliability code" is a code to indicate reliability level of the direct binding for each biomolecule pair and/or the experimental method whereupon the direct binding is proved.

"Connect search" means automatically searching a linkage of functionally or biosynthetically related molecules that include designated one or more arbitrary biomolecules or bio-events.

Connect search may be carried out with filtering of biomolecule pairs by combining one or more information items such as a relation code, a relation-function code, a reliability code, an acting organ, or directionality of a biomolecule pair. Furthermore, it is possible to search a desirable molecule-function network by scoring the searched molecule linkages while combining one or more information items such as a relation code, a relation-function code, a reliability code, an acting organ, or directionality of a biomolecule pair.

Connect search can be carried out by designating an arbitrary item such as a subnet, a pathological event, a drug molecule, a gene and information related to a disease, similarly to the case where a biomolecule or a bio-event is designated.

"Molecule-function network" means a linkage of functionally or biosynthetically related molecules obtained as a result of a connect search, by using a biomolecule-linkage database, wherein one or more items selected arbitrarily from biomolecules, bio-events, subnets, pathological events, drug molecules, or genes are designated.

"Drug molecule" means a molecule of a compound manufactured and used for medical treatment as a drug, and also includes a compound with known physiological activity such as a compound used for medical and/or pharmaceutical research and a compound described in patents or literatures.

"To correlate with information on bio-event" means to indicate or discover that the expression of a certain bioevent is related to a certain biomolecule, a drug molecule, genetic information, or a molecule-function network.

"Molecule annotation" is information in a database added to data on the molecule such as a biomolecule and a drug molecule.

"Categorization" means classifying information on biomolecules, biomolecule pairs, bio-events and others into predetermined categories and describing said information with notations representing the pertinent categories, instead of storing the given information intact, when the information is stored into a database. The aforementioned examples in "structure code", "function code", "relation code", and "relation-function code" are the examples of "categorization".

"Originating organ" means an organ, tissue, region in an organ or tissue, a specific cell in an organ or tissue, region in a cell and others, where a biomolecule is originated.

"Existing organ" means an organ, tissue, region in an organ or tissue, a specific cell in an organ or tissue, region in a cell and others, where a biomolecule is stored after its generation.

"Acting organ" means an organ, tissue, region in an organ or tissue, a specific cell in an organ or tissue, region in a cell and others, where a biomolecule or a key molecule causes a bio-event.

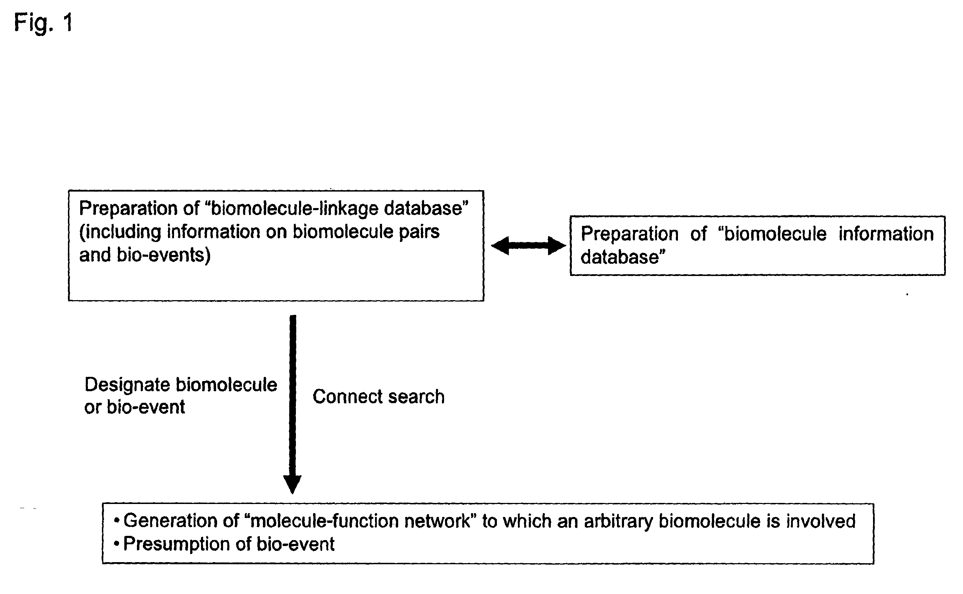

As one of the embodiments of the present invention, the following method is provided (Fig. 1). First, a "biomolecule-linkage database" storing the information on pairs of direct-binding biomolecules is prepared. Information on biomolecules themselves such as a molecule ID of a biomolecule may be included here, however, it is desirable to store them in a separate database, a "biomolecule information database". Next, one or more arbitrary molecules are designated from the aforementioned "biomolecule-linkage database" and a connect search is carried out to obtain a "molecule-function network" which is a representation of the functional or biosynthetic linkage of one or more biomolecules.

By correlating information on bio-events to at least those biomolecule pairs consisting of a key molecule and its target biomolecule among biomolecule pairs, it is possible to presume, together with the "molecule-function network", bio-events to which molecules in the molecule-function network are directly or indirectly related. Furthermore, by adding information on the relation between a quantitative or qualitative change of a key molecule and up-or-down of a bio-event, it is possible to presume whether a quantitative or qualitative change of an arbitrary molecule on the molecule-function network works for exaltation / increase of a bio-event or for suppression / decrease of a bio-event.

A principal role of the "biomolecule information database" is to define a molecule ID or an ID to the formal name of each biomolecule, and it is desirable to store necessary information on biomolecules themselves. For example, it is desirable to store information on molecule name, molecule ID, structure code, function code, species, originating organ, existing organ and others. Furthermore, even for a biomolecule that is not isolated experimentally nor confirmed to exist, one may assign a temporary molecule ID and other information, for example, to a molecule whose existence is predicted from experiments with other species.

Information on amino acid sequence and/or structure of each biomolecule may be included in the "biomolecule information database", however, it is desirable to store said information in a sequence database or a structure database and take out the information based on the molecule ID as necessary. For those with low molecular weight among biomolecules, it is desirable to store not only the formal molecule name but also the data necessary for drawing a chemical structure in the biomolecule information database or a separate database, so that chemical structures can be appended to the representation of the molecule-function network as necessary.

If it is more convenient to treat multiple biomolecules collectively, for example, if two or more biomolecules exhibit activity or function as an oligomer or as a group, it is acceptable to define them as one virtual biomolecule and register it in the "biomolecule information database" assigning a molecule ID. In this case, it is preferable to assign and register a molecule ID to each constituting molecule, and set up in the record of the virtual biomolecule, a field which describes molecule IDs of the constituting molecules, if the constituting molecules are known. Even if the constituting biomolecules are unknown, it is possible to define a virtual biomolecule having a specific function as a group, and use it for the definition of a biomolecule pair.

If a biomolecule consists of two or more domain structures, and if it is judged to be more favorable to treat each domain independently for such a reason that the respective domains have different functions, it is acceptable to treat each domain as an independent molecule. For example, it is preferable to give a molecule ID to each domain and register it in the biomolecule information database together with the original biomolecule. By setting up a field describing molecule IDs of the divided domains in the record of the original biomolecule, it is possible to describe that one biomolecule has two or more different functions. When a specific sequence on the genome sequence that does not correspond to a gene has a certain function or is recognized by a specific biomolecule, it is possible to treat the part of the sequence as an independent biomolecule and assign a molecule ID for defining a biomolecule pair.

Information on the biomolecule pair is stored in the "biomolecule-linkage database." For each biomolecule pair, molecule IDs of two biomolecules forming the pair, a relation code, a relation-function code, a reliability code, bio-events, acting organs, conjugating molecules, and other additional information are registered. For a molecule pair of a key molecule and its target biomolecule, it is desirable to input bio-events, up-or-down information of bio-events corresponding to a quantitative or qualitative change of either molecule, pathological events and others as much as possible. For a biomolecule pair without a key molecule, it is desirable to input bio-events and pathological events when there are bio-events or pathological events to which said biomolecule pair is directly related. Up-or-down information of a bio-event corresponding to a quantitative or qualitative change of a key molecule may be described as simplified information such that the bio-event increases or decreases compared to a normal range corresponding to the increase of the key molecule, for example. When one enzyme catalyses reactions of two or more kinds of substrates and generates different reaction products respectively, a representation specifying the relation among the enzyme, substrate and reaction product may be added.

Since the "biomolecule information database" and the "biomolecule-linkage database" are different in their content and organization, they are treated conceptually as independent databases in the present description. However, it is needless to say that those two kinds of data may be stored in one database combining the both, in the light of the purpose of the present invention. In other cases, two or more "biomolecule information databases" and two or more "biomolecule-linkage databases" may exist. In this case, it is possible to use those databases by selecting and combining them appropriately. For example, data for different species discriminated by a specific field may be stored in the same "biomolecule information database" and "biomolecule-linkage database", or alternatively, data for human and mouse may be stored in separate databases.

As "relation code", it is acceptable to input with words such that two molecules constituting a biomolecule pair are an agonist and a receptor, or an enzyme and a substrate, for example. However, it is desirable to input with categorization, for example, 10 for the relation between an agonist and a receptor, 21 for the relation between an enzyme and a substrate, 22 for the relation between an enzyme and a product. As "relation-function code", it is convenient to store the class of functions such as hydrolysis, phosphorylation, dephosphorylation, activation and inactivation, wherein it is also desirable to input them with categorization. Information on the relation code and the relation-function code may be used as a filter at the time of a connect search, for example, to consider only enzyme reactions, to ignore inactivating relations, or to ignore relations of induction or suppression of gene expression.

Relations between biomolecule pairs are not always clear as in the case of an enzyme and a substrate. There are cases in which mutual roles of both molecules are not clear, like two protein molecules judged to have protein-protein interaction by the two-hybrid experimental technique, for example. In order to carry out a connect search including such biomolecule pairs, it is convenient to treat whether the relation between two molecules constituting the biomolecule pair is directional or non-directional. To each biomolecule pair, it is desirable to use a relation code that can distinguish to which case it belongs. In the former case, the biomolecule pair is treated as having a fixed direction of action and only the input order of the two molecules in the representation of the biomolecule pair is considered at the time of search. Whereas in the latter case, the biomolecule pair is treated as having unknown direction of action and a relation with reverse direction is also considered. By giving directionality to a biomolecule pair like this, it becomes possible to designate a direction of search at the time of a connect search, for example, to search only an upstream direction or only a downstream direction in a molecule-function network.

There are various levels of information on direct-binding biomolecule pairs, from definite information that have been experimentally proved, to those tentatively assumed as biomolecule pairs. Furthermore, in some experimental methods, there are cases that some biomolecule pairs are included by mistake due to false positives. Consequently, it is desirable to add "reliability code" to information on each biomolecule pair, which indicates the reliability level and the experimental method. Reliability code can be used not only for presenting the information but also for the purpose of filtering, for example, that only those biomolecule pairs with the code beyond a certain level of reliability are taken into account. Furthermore, reliability code can be used for scoring a molecule-function network based on the code, and thereby reflecting the score on display of the result or reducing the range of the network to be displayed.

If information on the organs where a biomolecule is stored and information on the organs on which the biomolecule acts are stored, in addition to information on the organs where a biomolecule is generated, it becomes easy to describe, at the time of the generation of a biomolecule-function network, such a phenomenon that a molecule generated in a certain organ and going outside a cell acts on the target biomolecule on the membrane of other cell from outside. It is desirable to input information on the originating organs and the existing organs of a biomolecule in the "biomolecule information database", and to input information on the acting organs in the "biomolecule-linkage database." Here, the description of the originating organs, existing organs, and acting organs is not particularly limited to organs, and may include information on tissue, region of an organ or tissue, a specific cell in an organ or tissue, intracellular region and others. Information on the originating organ, the existing organ, and the acting organ can be used as a filter at the time of a connect search, for example, to select only those biomolecule pairs generated in the same organ for the search.

Any descriptions are acceptable for describing experimental or predictive methods that proved the direct binding, a kind of bio-event, up-or-down of a bio-event corresponding to a quantitative change of a key molecule, intracellular region, tissue, an organ, region in an organ, as long as the descriptions are simplified ones. However, it is desirable to categorize and convert the descriptions to short alphanumeric notations and others. If they are defined with a dictionary of synonyms, it is possible to process synonyms at the same time and to minimize mistakes at the time of input.

A concept of the "connect search" which generates a "molecule-function network" from the "biomolecule-linkage database" is shown in the following. Any method may be used for the "connect search" of the present invention, as long as this concept is realized. For example, an algorithm of "depth first search" described in Chapter 29 of "Algorithm in C" (Addison-Wesley Pub Co, 1990) by Sedgewick may be used.

If a biomolecule pair consisting of biomolecules represented by molecule IDs a~z is described like (n,m), a biomolecule-linkage database is described as a group of biomolecule pairs as follows.

(a, c) (a, g) (b, f) (b, k) (c, j) (c, r) (d, v) (d, y) (e, k) (e, s)
(g, u) (j, p) (k, t) (k, y) (p, q) (p, y) (x, z)

If a molecule-function network containing c and e is to be generated by a connect search, for example, biomolecule pairs (c, j) (j, p) (p, y) (y, k) (k, e) having one of the pair molecules in common are searched successively, and "c - j - p - y - k - e" which is a linkage of molecules c, j, p, y, k, e is obtained as a molecule-function network.

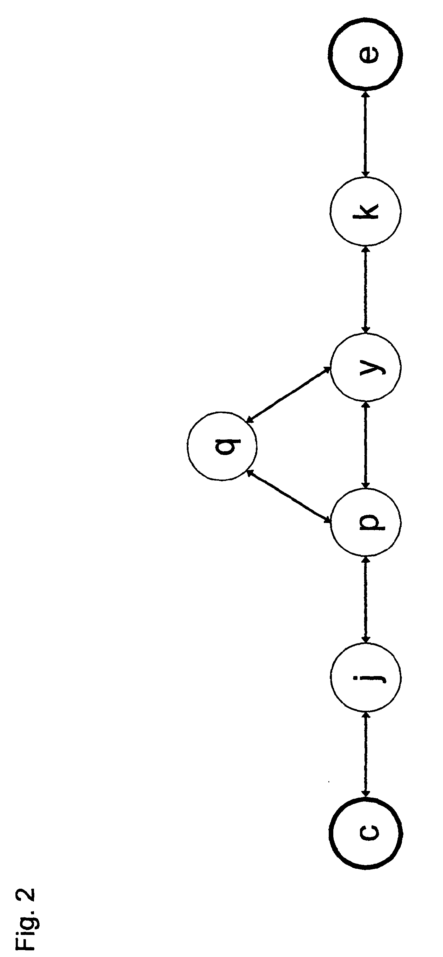

A molecule-function network obtained by a connect search is not always a linear paths as described above. For example, if a connect search is carried out designating c and e as search points when a biomolecule pair (q, y) has been further added to the above-mentioned biomolecule-linkage database, a molecule-function network shown in Fig. 2 is obtained. Here, the number of biomolecule pairs lying between c and e is 5 in the shortest path (through p - y), and 6 in the longest path (through p - q - y). The number of biomolecule pairs lying between two molecules in a molecule-function network is hereafter referred to as "path number."

At the time of a connect search, it is possible to restrict biomolecule pairs included in a molecule-function network with a path number. For example, biomolecule pairs may be restricted to those constituting a path with the minimum path number (c - j - p - y - k- e in the example of Fig.2), or the path number may be restricted within a certain range (for example, 5 to 6). By restricting the path number like this, it is possible to adjust the range of the molecule-function network generated by a connect search as necessary.

Based on the obtained "molecule-function network," it is possible to carry out presumption of bio-events as follows. If a biomolecule e is a key molecule and has information on a bio-event E, it is possible to presume that biomolecules c, j, p, y, k relate to the expression of the bio-event E directly or indirectly. Moreover, if there is information on up-or-down of a bio-event such that decrease of molecule e elevates the expression of bio-event E, it is possible to presume the effect of quantitative or qualitative changes of arbitrary molecules out of c, j, p, y, k to the expression of the bio-event E, considering relations of (c, j) (j, p) (p, y) (y, k) (k, e).

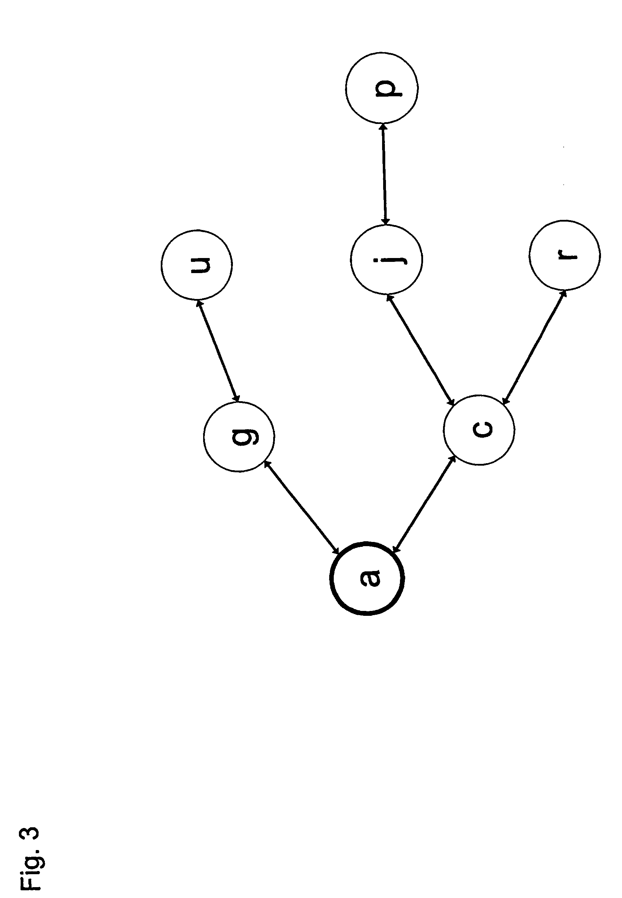

Connect search may be carried out by designating one biomolecule as a starting point. In this case, it is desirable to designate an upper limit of the path number between the starting-point biomolecule and one or more endpoint biomolecules as a termination condition of the search. For example, when a connect search is carried out using the aforementioned biomolecule-linkage database, while designating the biomolecule "a" as the starting point and 3 as the upper limit of the path number, a molecule-function network shown in Fig.3 is obtained.

Connect search can also be carried out by designating three or more multiple biomolecules. For example, when three biomolecules a, b, c are designated, connect searches are carried out for three pairs of a and b, a and c, and b and c, respectively; and suppose three sets of molecule-function networks

a-d-e-f-b
a-d-e-g-c,
b-f-e-g-c

are obtained. By obtaining a common part (AND operation) or a union (OR operation) of these sets or by obtaining a part common to two or more sets, it is possible to obtain a molecule-function network including three biomolecules a, b, c.

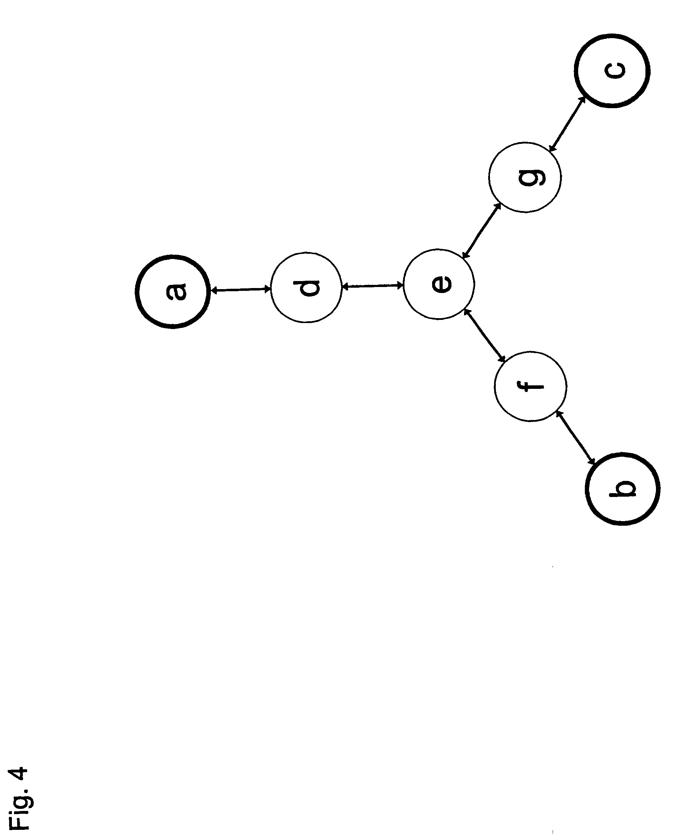

When all three biomolecules a, b, c have information on a bio-event E, it is possible to presume that a group of biomolecules a, b, c, d, e, f, g, which appear in the molecule-function network obtained as a union by the OR operation as shown in Fig.4, are related directly or indirectly to the expression of the bio-event E. Furthermore, it is possible to presume that a biomolecule "e", which appears in the molecule-function network obtained as a common part by the AND operation, may play an important role in the expression of the bio-event E with high probability.

Connect search can be carried out using the relation information between data items such as subnets, pathological events, drug molecules, genes, and information on diseases, similarly as in the above-mentioned example using information on biomolecule pairs. In this case, the aforementioned procedure may be carried out by treating two data items that are correlated in the relation information similarly to the two biomolecules in a biomolecule pair. By this way, it becomes possible to generate a molecule-function network while designating not only a biomolecule or a bio-event, but also a subnet, a pathological event, a drug molecule, a gene, or information on a disease.

Furthermore, it is possible to predict the effect on the amount of bio-event expression $Q_E$ given by N biomolecules on a molecule-function network from a certain biomolecule to a key molecule, by the following formula, for example. Here, $S_i$ is a qualitative evaluation value of the condition of the i-th biomolecule, $R_i$ is a value representing the amount of the i-th biomolecule, $V_i$ is an evaluation value of the environment where the i-th biomolecule exists, and f is a multiple-valued function with $3 \times N$ input values.

$$Q_E = f (S_1, R_1, V_1, ... S_N, R_N, V_N)$$

Whereas the kinds of bio-events relating to one molecule-function network is not limited to one and it is expected that there are several molecule-function networks related to one kind of bio-event, it is possible to screen related molecule-function networks from the side of bio-events. For example, if a "molecule-function network" containing enormous number of biomolecules is generated by designating one or more biomolecules, it is possible to screen the range of the "molecule-function network" by adding information on bio-events. As a matter of course, it is also possible to generate a "molecule-function network" with the condition that some kind of mediator molecule, or relation between said molecule and a target biomolecule is included.

It is possible to generate a molecule-function network within a necessary range by dividing, filtering, extracting a subset from, and/or hierarchizing the data of "biomolecule-linkage database" appropriately. Dividing, filtering, and extracting a subset can be carried out by search methods such as a search to the data items specific to the database of the present invention, a general text search using keywords, a homology search to amino acid sequences or nucleic acid sequences, a substructure search to chemical structures, a search designating the directionality of biomolecule pairs, a search using the data items specific to the database of the present invention for scoring according to certain criteria, and others. By carrying out these searches to the "biomolecule-linkage database" or the "biomolecule information database" beforehand, it is possible to generate a restricted molecule-function network or a characterized molecule-function network.

For example, it is possible to generate a "molecule-function network" with restricted range by generating a partial database screened from viewpoints such as biomolecules generated in liver and bio-events occurring in skin using the information on originating organs or acting organs, and carrying out a connect search. It is possible to generate a molecule-function network with desirable characteristics or with desirable range by dividing, filtering, and/or extracting subset of the molecule-function network generated by a connect search, carrying out the aforementioned search to biomolecules or biomolecule pairs included therein. Furthermore, connect search may be carried out while restricting the direction of the network by designating the directionality of biomolecule pairs, or a molecule-function network obtained as a result of a connect search may be screened by scoring based on the reliability code. Such restriction and characterization not only facilitate the search, but also are effective for helping the user to understand the molecule-function network by highlighting a specific group of biomolecules or biomolecule pairs on the molecule-function network.

By dividing, filtering and/or extracting subset of the "biomolecule-linkage database" appropriately based on the linkage on the network, and by storing and using information indicating its inclusive relation, it is possible to hierarchize the "molecule-function network." Even when there are some unknown molecules or unknown linkages between molecules, it is possible to generate a tentative molecule-function network by combining them to one virtual biomolecule and defining a pair with other molecule. When an extremely complicated network is generated because of the enormous number of the molecules included therein, it is possible to describe the network simply by defining two or more biomolecules linked in the network as one virtual biomolecule.

Use of such hierarchies makes it possible to speed up a connect search, and to avoid extreme complexity appropriately by making precision of the network description adjustable. In the present description, such a partial network consisting of two or more biomolecule pairs linked in the network is called a "subnet".

Any partial network can be designated as a subnet, however, it is convenient to treat cascade, pathway and/or cycle, which is well known to researchers like TCA cycle and pentose phosphate cycle in the metabolic system, as a subnet. Furthermore, a certain subnet may be included in a different subnet, for example, the metabolic system itself may be regarded as an upper subnet including multiple subnets.

Although there is a method of treating each subnet as one virtual biomolecule, it is convenient to store information on biomolecule pairs constituting a subnet and information on the hierarchy of the subnet in the "biomolecule-linkage database". Moreover, one may set up an upper data hierarchy to represent a subnet in the "biomolecule-linage database" and store therein the information on said subnet. The hierarchization of biomolecule pairs by subnet is not limited to two layers, and one may store a group of multiple subnets as a still upper subnet. In order to facilitate cross-referencing between the molecule pair data and the upper-hierarchy subnet data at the time of the network generation, it is desirable to store information indicating mutual relation between molecule pair and subnet, respectively in the molecule pair data and in the subnet data. It is needless to say that one biomolecule pair may be related to multiple subnets.

It is desirable to include in the subnet data of the hierarchized "biomolecule-linkage database", not only the links to biomolecule pairs in the lower hierarchy but also the information on relation between subnets. For example, glycolytic pathway and TCA cycle are subnets working in order in the metabolic system, and it is possible to store the relation between these subnets as a pair in the upper hierarchy. In this case, it is desirable to add information on biomolecules that become contact points between the subnets as well as the information on the subnet pair.

Furthermore, one of the characteristics of the present invention is that biomolecules themselves as well as the networks can be hierarchized, and that the hierarchized information can be stored and used in the "biomolecule information database". For rapidly searching and conveniently and variously displaying a network, it is desirable to hierarchize both information on biomolecules and information on biomolecule pairs. Items to be hierarchized for biomolecules can be exemplified as follows. Among biomolecules, there are cases in which multiple different molecules gather specifically to express a certain function, and there are also many cases in which appearance or type of functions are controlled depending on the difference in complexation states of molecules. Furthermore, as observed in immunocytes, there are cases in which relations to bio-events or cell functions are determined by the combination of multiple molecules expressed on the cell surface. In such cases, there is a method of treating the complexation state of molecules as one virtual biomolecule as described above, but as another method, an upper data hierarchy which represents the complexation state of molecules may be defined in the "biomolecule information database" and the information on said complexation state may be stored therein. In order to facilitate cross-referencing between the biomolecule data and the upper hierarchy data at the time of generating a molecule-function network, it is desirable to store information representing mutual relation between the biomolecule data and the upper hierarchy data, respectively in the biomolecule data and in the upper hierarchy data. It is needless to say that one biomolecule may be related to multiple upper hierarchy data.

As one of the other methods of treating the complexation state of biomolecules with hierarchization, it is acceptable to setup a field that registers respective molecules participating in the complex in the lower hierarchy of the molecule in the biomolecule-information database. In this case, it is preferable to register biomolecules in a complexation state as one virtual biomolecule, and register molecule IDs of respective molecules in the lower-hierarchy field that is setup as above.

Among biomolecules, there are those whose functions are changed by modification such as an enzyme reaction. Examples of such modification include phosphorylation, methylation, acetylation, ubiquitination, and their reverse reactions. Biomolecules with different modification states may be discriminated by giving separate molecule IDs, however, it is more convenient to setup a data hierarchy indicating the information on modification in the biomolecule-information database. For example, by setting up a field indicating "modification state" in the biomolecule-information database and by describing data such as "phosphorylation", it becomes possible to discriminate different modification states of a molecule while treating the molecule with the same molecule ID. When such hierarchization by the modification state is introduced, the molecule may be treated by a combination of the molecule ID and the data in the "modification state" field.

The aforementioned hierarchization can be extended besides the modification state of a biomolecule. For example, when affinity or reactivity of a biomolecule to other biomolecule changes according to the structural change of the biomolecule or combinations of multiple modification sites, it is recommended to discriminate such a change of state between active type and inactive type, for example, and store the data in the lower hierarchy of the molecule data.

Among biomolecules, there are those that bind or do not bind to other biomolecules depending on the change of state such as the modification state or the active type versus the inactive type. In such a case, the biomolecule may be treated differently based on the above-mentioned hierarchized molecule data, and biomolecule pairs may be registered in the biomolecule-linkage database for respective states of the biomolecule. As another method, a biomolecule pair may be registered in the biomolecule-linkage database based on the molecule IDs only, and the states of the two molecules that lead to binding and/or reaction may be registered as additional information to the biomolecule pair.

Among bio-events and pathological events, there are many that cannot be related to a specific biomolecule pair. For example, there are cases in which a relation between a bio-event or pathological event and formation of a certain subnet is known, but the biomolecule pair to which said event is directly related is unknown. In such cases, it becomes possible to describe the relation between said event and the biomolecule network by relating the bio-event or pathological event to the subnet data which is the upper hierarchy of the biomolecule pair, using the aforementioned hierarchization of biomolecule pair data.

If a complexation state of specific molecules or an expression state of certain molecules on cell surface is related to an expression of a certain bio-event or pathological event, it is possible to describe the relation between said event and the biomolecule network by relating the bio-event or pathological event to the complexation state of molecules or the expression state of molecules using the aforementioned hierarchization of complexation states of molecules or expression states of molecules.

Among bio-events and pathological events, there are some that can be related neither to a specific biomolecule pair nor to a subnet. An example of such cases is a pathological event "inflammation" which is caused by combination of various bio-events such as the release of inflammatory cytokines, infiltration of leukocytes to tissue, and increase in permeability of capillary vessel. In order to handle such an event, it is preferable to hierarchize bio-events and pathological events, describe events that can be related to biomolecule pairs and subnets in the lower hierarchy, and describe event that occurs in relation with the events in the lower hierarchy in the upper hierarchy. It is needless to say that more than two levels of hierarchy may be used this hierarchization. In order to facilitate cross-referencing between events at different hierarchies, it is desirable to store the information that indicates relations to the data in the upper and lower hierarchies, in the event data of respective hierarchies. By such hierarchization of data of bio-events and pathological events, it becomes possible to describe the relation with molecule-function networks for those events that cannot be related directly to a specific biomolecule pair or a subnet.

As another embodiment of hierarchization of bio-events and pathological events, bio-events and pathological events may be classified to multiple groups and/or hierarchies comprising up-or-down of the key molecule, biological response, symptom and syndrome, information on clinical marker value, disease and complication and others. For example, when a certain relationship is observed in a certain disease for a pair of bio-events and/or pathological events that are classified into groups or hierarchies as mentioned above, it is recommended to describe the relation between said events. By this method, the relation between a disease and a molecule-function network can be described easily via the up-or-down information of the key molecule.

As exemplified above, by hierarchizing and storing the data in "biomolecule information database" and "biomolecule-linkage database," it becomes possible to carry out the generation of molecule-function networks effectively corresponding to various purposes.

There are cases wherein the same bio-event or pathological event needs to be correlated to different biomolecule pairs or subnets depending on the condition such as normal versus disease states or different disease states. In such a case, it becomes possible to search two or more molecule-function networks to which the same bio-event or pathological event is related, by correlating the event to different biomolecule pairs or subnets.

When a relation between a certain biomolecules (molecule A) in the glycolytic pathway and a certain protein (molecule B) in a certain kinase cascade is examined, it is necessary to carry out a connect search with enormous number of molecule pairs if the data without hierarchization are used, and the search is practically impossible when the path between molecule A and molecule B is too long. On the other hand, using the hierarchized data, it is possible to carry out a connect search between the subnet "glycolytic pathway" and the subnet "certain kinase cascade" in the upper hierarchy, namely subnets, and if a path is found in the upper hierarchy, it is possible to carry out a connect search in the lower hierarchy of each subnet on that path as necessary. Thus, by dividing a pathway search problem to the problems in different hierarchies, it becomes possible to generate a molecule-function network that was impossible without hierarchization. In the connect search using the hierarchized data, it is also possible, similarly to the connect search using the data without hierarchization, to screen the data to be used for the search based on directionality of the relation between subnets or by scoring of the data items specific to the database of the present invention per se or according to a certain criteria.

If a specific subnet is frequently referred to in a connect search using the aforementioned hierarchized data, it is recommended to carry out a connect search beforehand within said subnet, and store the information on the molecule-function network in said subnet. With this process, it becomes possible to generate the entire molecule-function network more effectively.

Furthermore, when a molecule-function network related to the pathological event "inflammation" is generated, for example, it becomes possible to generate a more extensive molecule-function network by searching events in lower hierarchy related to the event "inflammation" of upper hierarchy, and by carrying out connect searches starting from biomolecule pairs or subnets to which said events of lower hierarchy are related.

As described above, by the present invention, it is possible to generate molecule-function networks relating to arbitrary molecules based on the information on relations of direct-binding biomolecules, and to presume easily the bio-events and pathological events that are related directly or indirectly. Furthermore, the present invention can be used inversely for the purpose of selecting a molecule-function network with high possibility of relation with a disease based on the characteristic findings in the disease such as bio-events, pathological events and changes in the amounts of biomolecules, and predicting molecular mechanism of the disease. Moreover, by the present invention, it becomes possible to construct strategies for drug development such that inhibition of which process in the network is effective for treatment of a specific disease or a symptom, which molecule in the network is promising as a drug target (a protein or other biomolecule to be targeted in drug development), what kind of side effects are expected from the drug target, and what kind of assay system is appropriate for selecting drug candidates while avoiding the side effects.

In general, a drug molecule exerts its pharmacological activity by binding to a biopolymer such as a protein in an organism and by controlling its function. The actions of drug molecules have been studied more precisely compared to the actions of biomolecules, contributing to the elucidations of molecular mechanisms of target diseases. Thus, we noticed that the usefulness of the methods of the present invention is enhanced by adding pair relations between a drug molecule approved for manufacturing and used for medical treatment or a drug molecule used for pharmacological studies and its target biomolecule, to the aforementioned information on biomolecules and biomolecule pairs. In most cases, target biomolecules are proteins or proteins modified with sugars. It becomes possible to presume bio-events that are likely to lead to side effects based on the molecule-function network including the target biomolecule, and it also becomes possible to presume drug-drug interaction from crossovers in the molecule-function networks relating to drugs administered together. As a result, it becomes possible to select and determine dose of a drug while considering risk of side effects and risk of interaction between drugs.

Examples of the methods of the present invention wherein relations between a drug molecule and a target biomolecule are added are described below. A molecule ID is defined for the formal nomenclature of each drug molecule, and a "drug molecule information database" is prepared which stores all information on said molecule itself. For each drug molecule, the name, molecule ID, indications, dose, target biomolecules and other information are stored herein. As in the case of the biomolecule information database, information such as the chemical structure, amino acid sequence (in case of peptides or proteins) and steric structure of drug molecules may be included in the "drug molecule information database", but it is preferable to store them in a separate database. For the purpose of discriminating between drug molecules and biomolecules or between proteins and small molecules, it is acceptable to use discrimination by structure code and others, or to employ a rule of assigning molecule IDs wherein the first letter tells the difference, for example. Furthermore, if information such as the remarkable side effects, interaction with other drugs, and metabolizing enzymes are input from prescribing information or other literature about drugs, it will be helpful for the purpose of appropriate selection of a drug in relation to gene polymorphism based on the molecule-function network.

Furthermore, a "drug molecule-linkage database" which is a database containing the information on pairs of a drug molecule and a target protein as well as the information on their relation may be prepared. Molecule ID of the drug molecule, molecule ID of the target biomolecule, relation code, pharmacological action, indication and other information regarding the drug molecules are stored therein. Concerning the molecule IDs of the target biomolecules, it is necessary to use those defined in the biomolecule information database. Concerning data items common to the biomolecule-linkage database such as the relation code, it is preferable to use description rules conforming to those of the biomolecule-linkage database.

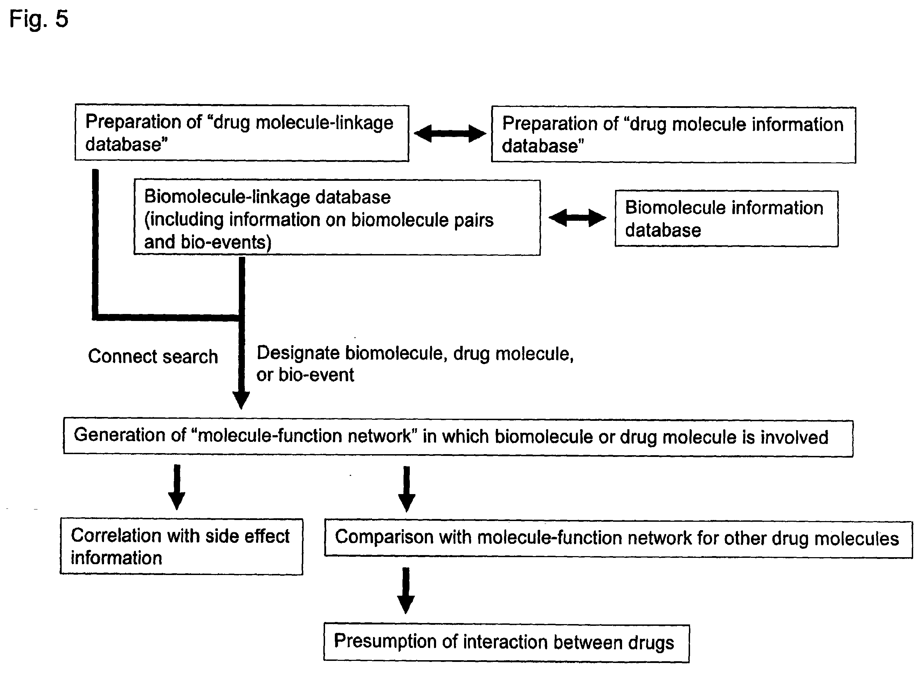

By preparing the "drug molecule information database" and "drug molecule-linkage database" and importing information on drug molecules and drug molecule pairs therein, the method of the present invention can be expanded as shown in Fig.5. Here, the generation of a molecule-function network and presumption of bio-events by a connect search can be carried out by a method similar to the aforementioned method wherein only biomolecule-linkage database and biomolecule information database are used, and information on known drug molecules that target molecules on said network is obtained as well. Furthermore, it is useful for the purpose of extracting a molecule-function network to which a designated drug molecule is related from the molecule-function networks that has been generated using only the biomolecule-linkage database and biomolecule information database.

On the other hand, elucidations of genetic information from various aspects are progressing rapidly including the analysis of human genome sequence. cDNAs are isolated in genome-wide scale, elucidations of orf (open reading frame) and gene sequences are progressing, and locating of genes on the genome is proceeding. Hereupon, as further embodiments of the present invention, the present invention can be expanded as follows by preparing a biomolecule-gene database which relates molecule IDs of proteins among biomolecules to the information of the genes coding said proteins comprising their names, abbreviated names, IDs and others. That is, by correlating genes and biomolecules, it becomes possible to understand the meaning of genes and proteins that are the markers of a disease and to understand the findings such as a relation between a disease and a gene polymorphism, in relation with molecules and bio-events in the molecule-function network. In the biomolecule-gene database, it is preferable to include information such as the amino acid mutation and abbreviation of gene polymorphism and relation with functions, as well as the species, location on the genome, gene sequence and function. It is acceptable to prepare two or more databases if necessary.

Based on the gene names located on genome sequences or the arrangement of genes, proteins that are translated by the action of a specific key molecule of the gene transcription mechanism such as a nuclear receptor are identified, and it becomes possible to reflect relations of mutual control between biomolecules on the molecule-function network. It is known that expressions of genes and proteins are different depending on organs. By the method of the present invention, importing such expression information into the "biomolecule information database" makes it possible to generate different "molecule-function network" for each organ, and it becomes possible, for example, to explain a phenomenon such that a drug molecule targeting a nuclear receptor exerts different or inverted actions in different organs. It is known that expressions of proteins change upon administration of a drug molecule. By interpreting the increase or decrease of amount of protein expression on the molecule-function network related to the target protein by the method of the present invention, it is useful for choosing drugs under consideration of the gene polymorphism.

In the aforementioned storage of information on gene transcription and protein expression, use of the concept of hierarchization also makes it possible to generate molecule-function networks more effectively and broadly. For example, for multiple genes and/or proteins that are transcribed or expressed by a specific nuclear receptor, it is preferable to set up the upper hierarchy representing the transcription of group of genes and/or expression of group of proteins in the "biomolecule information database" and to store the data of said group of genes and/or group of proteins therein. When there are bio-events and/or pathological events related to the transcription of said group of genes and/or expression of said group of proteins, describing relations between the upper hierarchy data of said group of genes and/or said group of proteins and said event in the "biomolecule-linkage database" makes it possible to generate molecule-function networks that cannot be described with the relation between individual gene or molecule and said event.

In the aforementioned method of hierarchical storage of information on gene transcription and protein expression, it is preferable to store the information as numerical parameters in the "biomolecule information database" if there is quantitative information on transcription or expression of respective genes or respective proteins. Using the numerical parameters, it becomes possible to describe the cases in which related bio-events and/or pathological events change depending on the differences of the amount of expression of individual gene or the amount of expression of individual protein.

Numerical parameters of gene transcription or protein expression may also be imported from an external file or database and used by the methods of the present invention, instead of storing them in the "biomolecule information database" as described above. In this case, it becomes easier to import the external data to the methods of the present invention by preparing a correspondence table between gene or protein IDs in the external data and molecule IDs in the "biomolecule information database" or gene IDs in the "biomolecule-gene database".

Diversity among individuals regarding genome and genes has been made clear. By linking such information to the methods of the present invention, it becomes possible to deepen the understanding about diversity among individuals and to realize medical treatment based on the diversity. For such gene polymorphism that a function of a specific biomolecule (protein) is impaired, interpreting it on the molecule-function network makes it possible to presume its influence on bio-events. It is advantageous for the understanding to link information on symptoms and abnormalities of bio-events in a genetic disease caused by a defect or an abnormality of a single gene to the methods of the present invention.

It has been reported in several typical diseases that there are several genes frequently observed in patients with the disease, namely disease-related genes. Supposing genetic habitus prone to suffer from a specific disease actually exists, there can be two or more molecule-function networks related to the adjustment of blood pressure, for example, and it is no wonder that considerable number of genes that might be related to the high blood pressure depending on the abnormality of any one of the molecules in any one of the networks. In order to interpret such a problem of polygenic genes, the methods of the present invention are indispensable.

Analyses of genomes and genes of animals such as mouse and rat have been progressing rapidly in recent years, and it is now possible to correspond those to human genome and genes. It is expected that proteins related to the regulation of physiological functions are considerably similar between these animals and human, however, the existence of appreciable differences has been an obstacle in drug developments. More cases are emerging in which proteins and protein functions are quite different between these animals and human, and it is useful for drug discovery to clarify the difference from the molecule-function network in human by linking them with the methods of the present invention. These methods are also useful for aiming at an appropriate use of animal drugs that have been switched in many cases from drugs originally developed for human.

If there is a disease model animal having similar pathological findings to a human disease, drug development is carried out with the pharmacological activities in that animal as indices, in many cases. Studies on genes of such disease model animals are also progressing, and relating them to the genetic information of human by the methods of the present invention will be helpful for elucidating a mechanism of said human disease.

For the purpose of elucidating a gene function, there are more and more cases where one creates a knockout animal in which a specific gene is disabled or a transgenic animal in which a gene is changed to the gene with weaker function or to the over expressing gene. There are many cases where the animals are lethal and unable to be born or no influence is found in the biological functions or behaviors. Even in the case where a certain abnormality is found in a newborn animal, it is believed to be very difficult to analyze the result of these animal experiments. In such experiments, it is convenient to carry out functional analyses after predicting influences of said gene operation using the methods of the present invention.

When methods of the present invention are used for the above-mentioned study on two or more species, it is preferable to prepare correspondences of genes or proteins between the species beforehand. As another method, data on respective species may be registered and used in the database of the present invention. In this case, it is preferable to store the information for discriminating the species in the "biomolecule information database" and in the "biomolecule linkage database". For example, by adding the species discriminator to the "originating organ", "existing organ" and "acting organ" items as an upper hierarchy, it becomes possible to register the species discriminator and use them in the methods of the present invention.

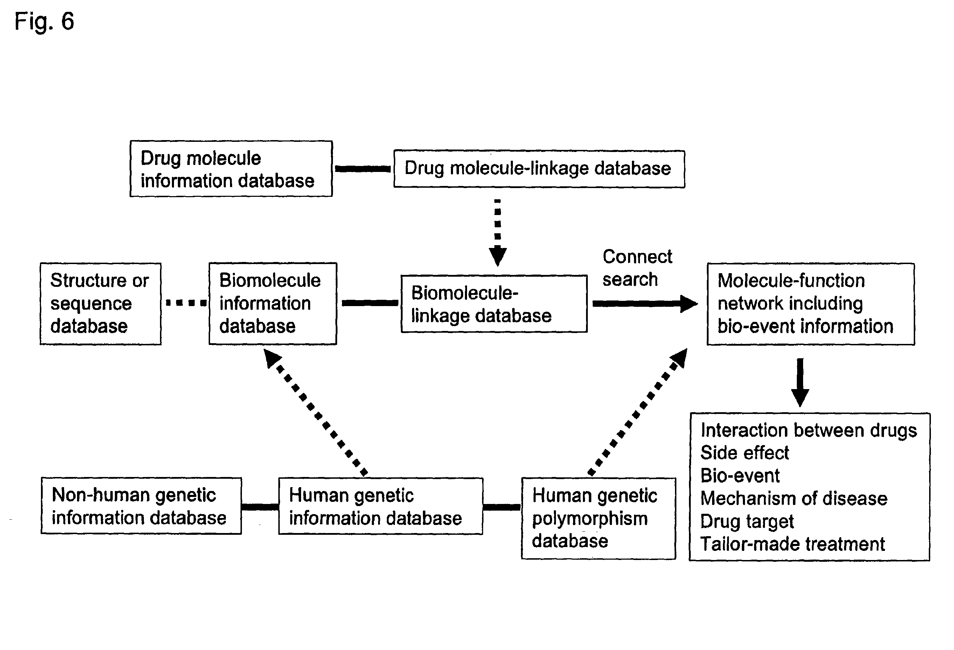

Attempts of integrating information related to genes from aspects of sequence IDs are progressing, along with the progress of genome analysis, and furthermore, attempts of locating genes on the genome sequence are also progressing. It is possible to construct an original genetic information database considering the cooperation with the aforementioned "biomolecule-linkage database" and use it for the aforementioned purpose. However, considering the fact that the information are enormous and tend to be open to public, it is highly possible that the aforementioned methods can be carried out by incorporating such public information into the methods of the present information pro re nata in the future (Fig. 6).

Biomolecule-linkage databases used in the methods of the present invention are not necessarily managed and/or stored at the same site. By unifying molecule IDs, it is possible to select appropriately one or more biomolecule-linkage databases managed and/or stored at different sites and use them by connecting with communication means and others. It is needless to say that similar disposition is possible not only for the biomolecule-linkage database, but also for the biomolecule information database, drug molecule-linkage database, drug molecule information database, gene information database, and pathology-linkage database used in the methods of the present invention.

As a still further embodiment of the present invention, there is also provided a method of preparing a database comprising information on biomolecules directly related to the expression of bio-events and said bio-events (a bio-event-biomolecule database) and using it with molecule-network databases that do not necessarily contain information on bio-events. As a still further embodiment, there is also provided a method of extracting a partial molecule network related to an arbitrary molecule from molecule-network databases that do not necessarily contain information on bio-events, and searching the aforementioned bio-event-biomolecule database based on the molecules constituting said network.

As a still further embodiment of the present invention, there is provided a method of searching data items in "biomolecule information database", "biomolecule linkage database", "drug molecule information database", "drug molecule-linkage database", "biomolecule-gene database", "pathology-linkage database" and others based on keyword and/or numerical parameter and/or molecular structure and/or amino acid sequence and/or base sequence and others, and generating a molecule-function network based on the result of said searching. Examples of generating a molecule-function network based on the search are described below, however, it is needless to say that the scope of the present invention is not limited to these examples.

In each database, various information such as molecule names, molecule IDs, species, originating organs and existing organs are stored as texts. By searching through these texts based on the complete match or partial match of character strings, it is possible to screen biomolecules, biomolecule pairs, bio-events, pathological events, drug molecules, drug molecule-biomolecule pairs, gene-protein correspondence data and others. Based on these screened information, it is possible to define one or more starting points and/or end points of a connect search or limit molecule pairs used in the connect search, making it possible to generate an appropriate molecule-function network according to its usage.

When chemical structures and/or steric structures of drug molecules are stored in the "drug molecule information database", carrying out a search based on full-structure match or sub-structure match or structure similarity makes it possible to screen drug molecules. Based on the screened drug molecules, it is possible to generate a molecule-function network related to said drug molecules and search bio-events and/or pathological events related to said drug molecules.

When numerical parameters such as those of gene transcription and protein expression are stored in the "biomolecule information database," carrying out a search based on these numerical parameters makes it possible to generate a molecule-function network corresponding to the amounts of gene transcription and/or protein expression. The search based on the numerical parameters can be carried out similarly, when the numerical parameters are imported from an external file or a database.

When amino acid sequences of proteins are stored in the "biomolecule information database" or in a related database, carrying out a search based on sequence homology or match of partial sequence pattern to these amino acid sequences makes it possible to screen biomolecules and generate a molecule-function network based on said biomolecules. This method is effective, concerning a protein with unknown function or its partial sequence information, for predicting a molecule-function network with which said protein fairly possibly has relation and for further predicting functions of said protein.

When base sequences of genes corresponding to proteins are stored in the "biomolecule information database", "biomolecule-gene database" or a related database, carrying out a search based on sequence homology or match of partial sequence pattern to these base sequences makes it possible to screen biomolecules and generate a molecule-function network based on said biomolecules. This method is effective, concerning a gene with unknown function or its partial sequence information, for predicting a molecule-function network with which a protein translated from said gene fairly possibly has relation and for further predicting functions of said protein.

As a further embodiment of the present invention, there is provided a method of generating or searching a molecule-function network using a "pathology-linkage database" that stores pathological events, biomolecules, and other information related to a certain disease (hereafter referred to as a "focused disease") with grouping and/or hierarchization. In the following, examples of preparing and using the pathology-linkage database are shown, however, it is needless to say that the scope of the present invention is not limited to the specific methods of grouping and hierarchization described below.

The pathology-linkage database stores pathological events, biomolecules, and other information that are related to the focused disease with grouping and/or hierarchization. Furthermore, when two or more data items belonging to any one of the groups have a relation in a certain focused disease, information on the relation between said data items is stored.

Key molecule
Biological response
Symptom and syndrome
Clinical marker value
Disease and complication

In the "key molecule" group of the pathology-linkage database, information on biomolecules for which quantitative and/or qualitative fluctuation is observed related to the disease is stored. By using the information on the key molecule, it becomes possible to search a molecule-function network from a pathological event, or search relation between a molecule in a molecule-function network and a pathological event.

Qualitative or quantitative fluctuation of a key molecule can be described as shown in the following examples. The case wherein a molecule A fluctuates to an increasing direction in a certain disease is described as A(+). Examples of such cases include an exaltation of enzyme activity or receptor activity. In the reverse, the case wherein a molecule A fluctuates to a decreasing direction is described as A(-). This case may include the case wherein the amount of the molecule is almost zero and may also include the case of a loss of function of the molecule. For example, this description can also indicate a phenomenon wherein a gene is not expressed due to an abnormality of methylation of the CpG island in the DNA promoter region, and accordingly, a protein that should have been transcribed, translated, and supposed to function is inactivated. Furthermore, use of this description makes it easy to presume a change in a molecule-function network when a specific gene is knocked out and the protein coded by that gene is deprived. The case wherein a certain molecule changes qualitatively in a certain disease is described as A(m). Such a case is often related to a mutation of the gene coding that protein.

In the "biological response" group of the pathology-linkage database, information on a biological response and a physiological phenomenon in an abnormal or excessive (or insufficient) condition for an organism, which is supposed to relate to a disease directly or indirectly is stored. A data item described in the biological response group may be correlated with a bio-event, a biomolecule pair, a subnet, and others in a molecule-function network. Here, a biological response mainly indicates a phenomenon at the level of molecules (including protein, enzyme activity, and gene), cells, and organs. Furthermore, the information may be described hierarchically. Moreover, a biological response may be hierarchized based on the information on the site or condition in which it occurs.

In the "symptom and syndrome" group of the pathology-linkage database, a pathological event characterizing the focused disease or evidencing a diagnosis of the focused disease is stored. It is preferable to describe a pathological event corresponding to a side effect of a drug molecule in this group. Furthermore, among the pathological events stored in this group, for those events that correspond or relate to a mechanism or an indication of a certain drug molecule, it is recommended to describe the events as data of said drug molecule in the "drug molecule information database." By this way, it becomes possible to search a molecule-function network even for a drug molecule whose target biomolecule is unknown.

In the "clinical marker value" group of the pathology-linkage database, test items that are actually used clinically or that might be used are stored. If a biomolecule tested in a clinical examination has been stored in the biomolecule information database, information that said molecule is tested in the clinical examination may be added as annotation of the biomolecule. By this way, it becomes possible to search a molecule-function network related to a biomolecule stored in the clinical marker value group. Furthermore, when a biomolecule in a molecule-function network is a target of a clinical examination, the information can be used for highlighting said biomolecule.

In the "disease and complication" group of the pathology-linkage database, information such as a name or a classification based on the onset mechanism, stage, and position of the lesion of the focused disease, and a complication related to the focused disease is described. Data items in the "disease and complication" group may be stored hierarchically, for example, based on the focused disease, stage, and position of the lesion.

It is preferable to describe terms in the pathology-linkage database according to a standard dictionary such as MedDRA/J (Medical Dictionary for Regulatory Activities Terminology; Society of Japanese Pharmacopoeia). Furthermore, it is recommended to add analogous words or synonyms in addition to the terms included in the dictionary.

By using the pathology-linkage database in combination with other databases of the present invention, it becomes possible to search molecule-function networks as shown below.

As mentioned above, when a target biomolecule to a certain drug molecule is described, it is possible to search a molecule-function network in which said drug molecule is involved by carrying out a connect search from said drug molecule through the target biomolecule. When the pathology-linkage database is used, it becomes possible to search a molecule-function network in which a drug molecule is involved even for a drug molecule with unknown target biomolecule, as follows.

For a drug molecule with unknown target biomolecule, corresponding items in the pathology-linkage database are searched based on information such as the applicable disease, indication, and mechanism of said drug molecule. Next, by carrying out a connect search between the data items of the pathology-linkage database using the relation information in the pathology-linkage database, information on the key molecule that could be linked to a molecule-function network is obtained. By carrying out a connect search using the biomolecule-linkage database from said key molecule, it is possible to search a molecule-function network in which said drug molecule is involved. If a corresponding item is found in the "symptom and syndrome" or "disease and complication" groups by searching the pathology-linkage database based on information on the side effect of a certain drug molecule, it is possible to search a molecule-function network related to the side effect of said drug molecule by carrying out a connect search from the item similarly as above.

The database and the computer system provided by the present invention can also be used as an input-output device for a user to add, edit, and delete data easily. The data edited by the user can be stored, and also can be merged with the previously provided data if necessary. Furthermore, the merged data can also be used for searching, reading, and the like. For example, a user can generate a novel molecule-function network by a connect search, or obtain novel event information pertaining to a molecule-function network, by adding, editing, and deleting data based on the biomolecule, biomolecule pair, bio-event, pathological event, information on drug molecule, gene, and other information that have been confirmed or predicted by experiments and others. Herewith, it becomes possible to presume a novel molecular mechanism of a disease. The data edited by the user may be recorded in the form of addition and/or substitution to the data items of the previously provided database, or the data may be managed independently as a file or a database for editing and may be used together with the data in the previously provided database as necessary.

It is possible to import the whole or a part of the biomolecules, biomolecule pairs, bio-events, pathological events, drug molecule information, and other information that are stored in external databases other than the databases of the present invention into the databases of the present invention.

As a still further embodiment of the present invention, there is provided a method of highlighting an item corresponding to any one of the items in the databases of the present invention or any one of the items on the generated molecule-function network when said item exists in the information being read on the terminal. With this method, it becomes possible for a user to know that said item is already registered in the database without searching the database. Furthermore, at this time, it is possible not only to highlight said item but also to display a part or a whole of the data items in the related database or the molecule-function network. Furthermore, it is also possible to carry out a connect search based on said item, and to generate and display a molecule-function network in which said item is involved.

As still further embodiments of the present invention, there are provided a computer system consisting of programs and databases to carry out the methods of the present invention; a computer-readable medium storing programs and databases to carry out the methods of the present invention; a computer-readable medium storing databases to be used by the methods of the present invention; a computer-readable medium storing information on the molecule-function networks generated by the methods of the present invention.

Characteristics of the methods of the present invention are as follows. By accumulating information on direct-binding biomolecule pairs having information on bio-events, a database of relations between molecules in an organism is generated. By a connect search to the aforementioned database which is a collection of parts, a molecule-function network related to one or more arbitrary biomolecules or bio-events is generated.

Based on the molecule-function network, bio-events to which one or more arbitrary molecule is directly related are presumed.

From the molecule-function network with information on one ore more bio-events, a mechanism of a disease, a possible drug target, a risk of a side effect and others are presumed.

From quantitative or qualitative changes of biomolecules, up-or-down of one ore more bio-events are presumed.

A molecule-function network having information on originating organs, existing organs and acting organs of biomolecules.

Presumption of side effects and interactions between drugs using the drug molecule information and the molecule-function network.

Interpretation of changes of protein expression upon administration of a drug molecule on the molecule-function network.

Analyses of influences of gene polymorphism on the molecule-function network, disease-related gene and others by linking to genetic information.


Examples


In the following, the present invention is explained with examples more specifically, however, the scope of the present invention is not limited by these examples. By referring to the examples described in the specification of PCT/JP01/07830, it will become easier to understand the following examples.


Example 1


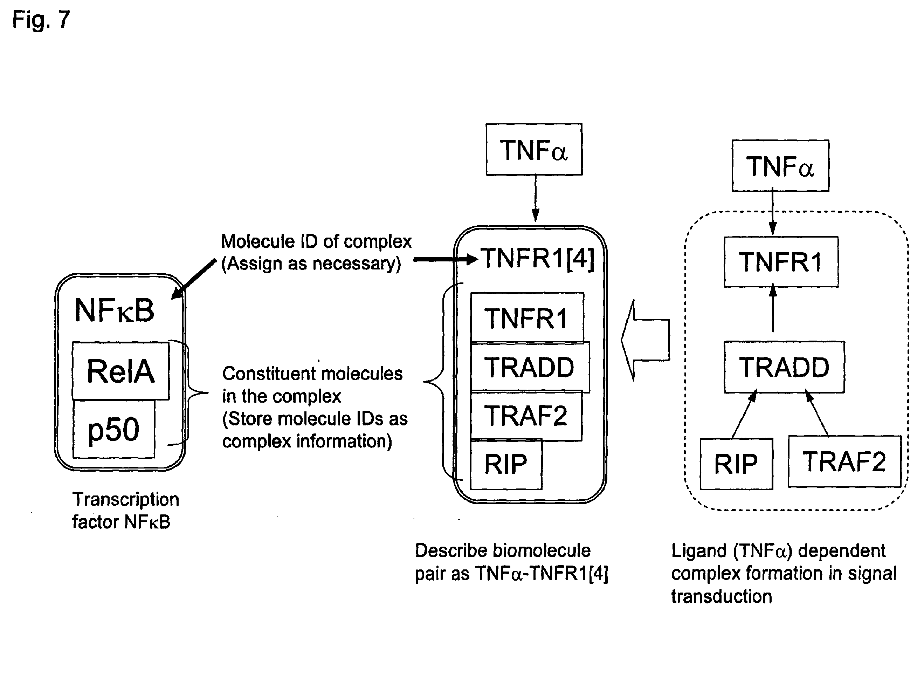

A method of registering and using information on a complex to the biomolecule-information database and biomolecule-linkage database when two or more biomolecules function as a complex (aggregate) is explained by taking the transcription factor NF κ B system and TNF receptor complex as an example (Fig. 7).

A transcription factor NF κ B functions as a hetero dimer of RelA and p50. Such molecules can be treated as one molecule in the following manner. In the biomolecule information database, setup a field "complex information" as an item of the molecule annotation, and register that the elements constituting NF κ B are RelA and p50. A complex formed in a ligand-dependent manner, for example, TNF receptor complex (TNFR1[4] in Fig.7), can also be treated as one molecule and thereby it becomes possible to describe a molecule-function network simply.

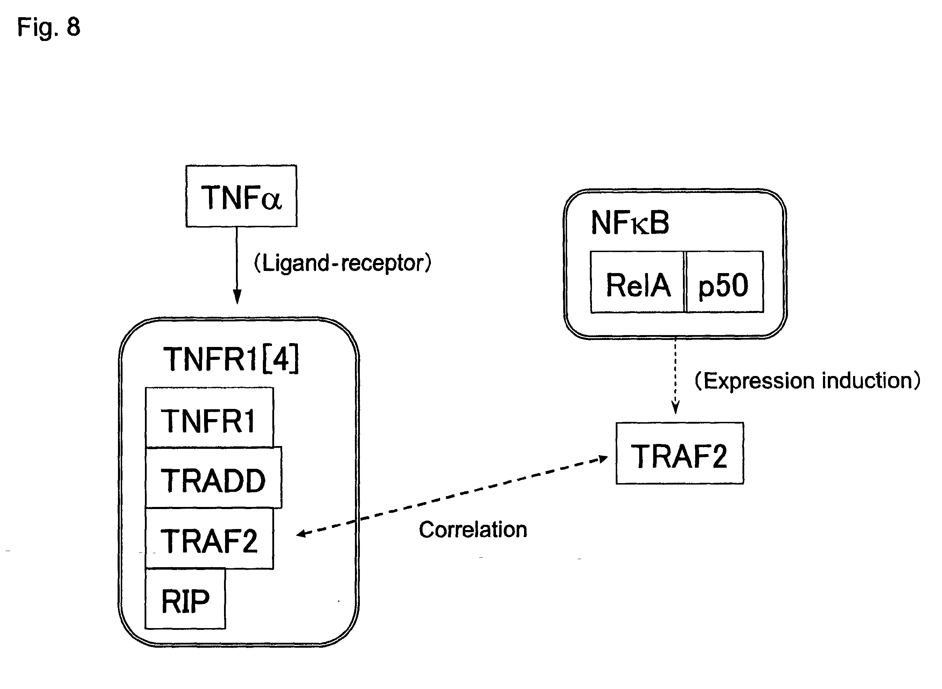

For some biomolecules, it is sometimes treated as a constitutive element of a complex, and in other cases it is treated as one independent biomolecule; for such biomolecules, both cases need to be considered. As shown in Fig.8, a biomolecule TRAF2 is treated as a constitutive element of a complex TNFR1[4], and treated as a single biomolecule whose expression is induced downstream of NF κB. In this case, by correlating the single biomolecule TRAF2 and the TRAF2 as a constitutive element of TNFR1[4], it becomes possible to describe a molecule network more precisely. Such a correlating procedure can be carried out automatically by matching the molecule ID in the molecule information database and the molecule ID in the complex information field.

Example 2

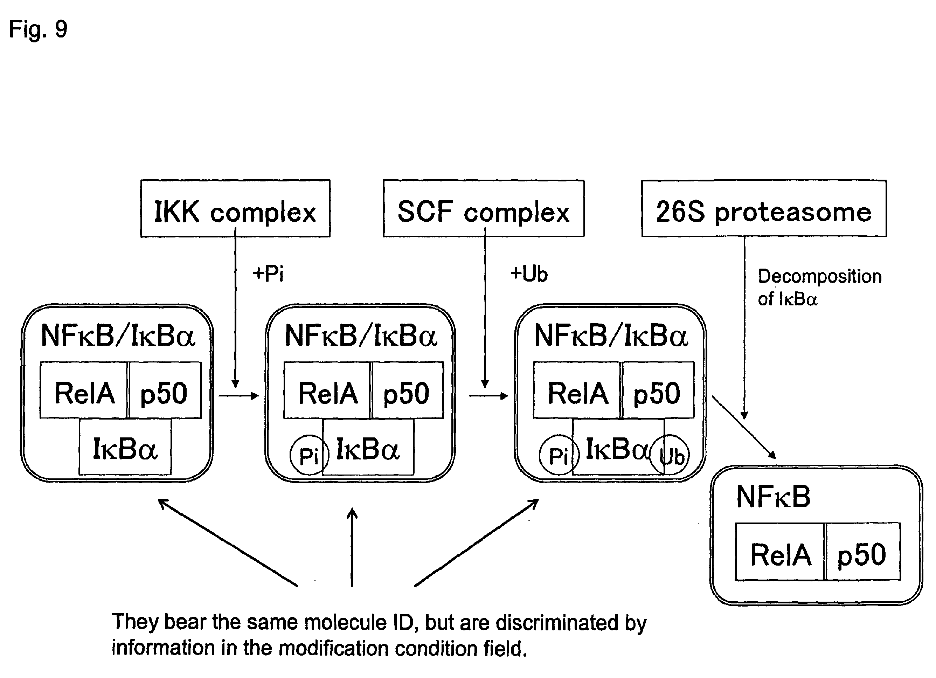

As an example of discriminating modification states of a biomolecule by hierarchizing the biomolecule data is shown for the NF κ B/I κ B α complex system (Fig.9).

NF κ B/I κ B α is first phosphorylated by IKK complex, then ubiquitinated by SCF complex which recognizes and binds to the phosphorylation site, and finally, I κ B α is decomposed by 26S proteasome. In this example, it is possible to treat NF κ B/I κ B α in unmodified state, phosphorylated NF κ B/I κ B α, and ubiquitinated NF κ B/I κ B α separately by the data in the hierarchy of "modification state." By using the hierarchized data like these, it becomes possible to describe relations between biomolecules such as IKK complex and NF κB/IκB α simply, without assigning different molecule IDs to different modification states of NF κB/I κB α.

Example 3

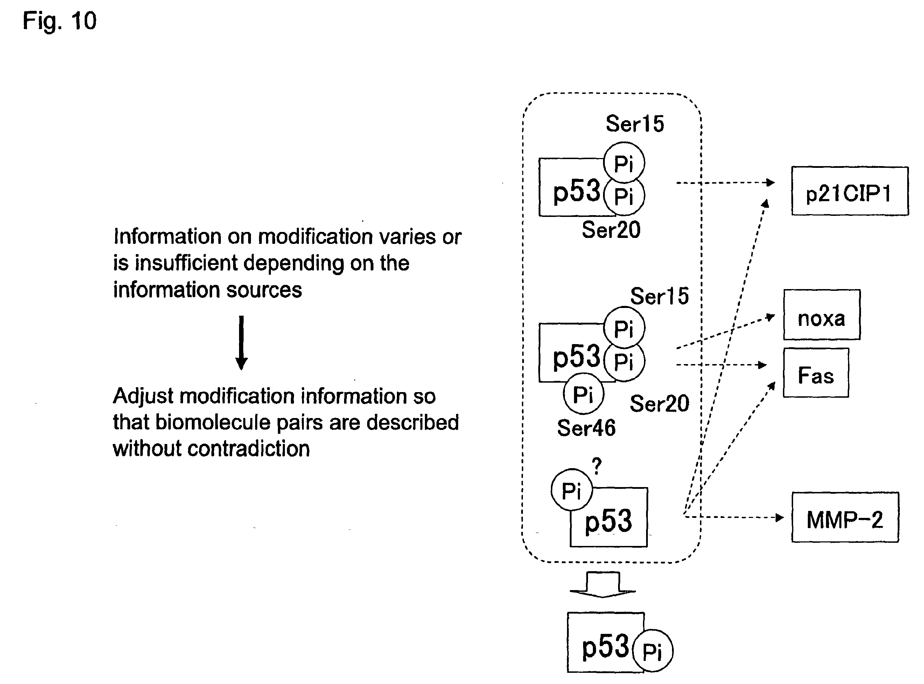

There are many cases where information on modification states of a biomolecule is only partially available and one cannot clearly discriminate among modification states in relation to the formation of biomolecule pair or to a bio-event. For example, in the system shown in Fig.10, relations between the biomolecule p53 where different sites are phosphorylated and partner biomolecules with which biomolecule pairs are formed are not clear. In such a case, the biomolecule in multiple modification states may be treated together as one entity.

Example 4

Regarding diabetes as the focused disease, an example of the data items included in the pathology-linkage database and an example of the data of correlation among them are shown.

Diabetes was classified into type 1, type 2, other special types, and pregnancy diabetes by ADA (American Diabetics Association) in 1997; however, it is understood as a multifactor disease where genetic factors and environmental factors are involved in a complicated manner.

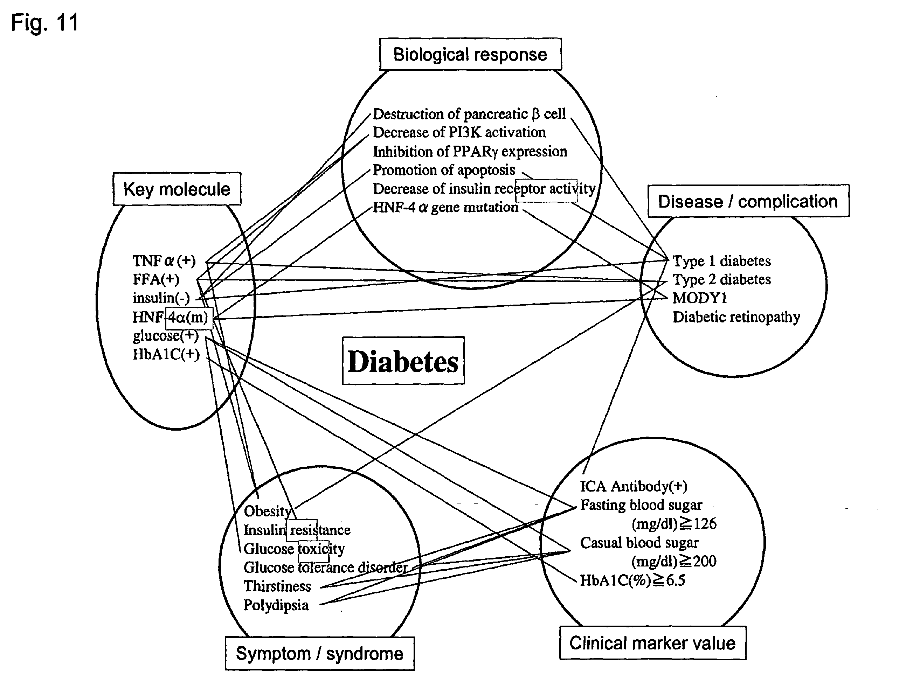

As an example of data items included in the pathology-linkage database, contents of the pathology-linkage database described by the method of the present invention regarding diabetes as the focused disease are shown in Table 1. In Fig.11, relations among the data items are shown conceptually. Each pair of data items linked with a line in the figure is stored in the pathology-linkage database as the relation information.

In the disease such as diabetes where many factors are involved, it is very difficult to schematize and understand the mechanism in a clear form; however, using the methods of the present invention, it is easily understood how respective data items of the pathology-linkage database classified into groups are related, and how relations between multiple data items are interrelated. When one tries to capture an overall picture or a specific picture of a complicated disease, it becomes possible to understand the disease from a point of view of relationship among the pathological events, biomolecules, biological responses and others that are related to the disease.

Table 1

| Key Molecule |
| --- |
| adiponectin(−) |
| b3−adR(−) |
| b3−adR(m) |
| FFA(+) |
| GIP(+) |
| GlcK(−) |
| GlcK(m) |
| D−Glc(−) |
| D−Glc(+) |
| HNF−1a(m) |
| HNF−1b(m) |
| HNF−4a(m) |
| insulin(−) |
| insulin(+) |

| Biological Response |
| --- |
| beta3 adrenergic receptor gene mutation |
| beta3 adrenergic receptor function decrease |
| Linkage with DRB1 gene locus |
| Dysfunction of GIP |
| Attenuation of insulin response to GIP |
| Increase in GIP secretion potency |
| Inhibition of GLUT4 translocation |
| Inhibition of GLUT4 expression |
| Linkage with HLA class II (DQA1) |
| Linkage with HLA class II (DRB1) |
| HNF−1alpha gene mutation |
| HNF−1beta gene mutation |
| HNF−4alpha gene mutation |
| IPF1 gene mutation |

| Symptom and syndrome |
| --- |
| Disorder in beta cell |
| Acidosis |
| Acetone odor |
| Insulin resistance |
| Familial hyperproinsulinemia |
| Pyogenic tendency |
| Itch |
| Kussmaul's respiration |
| Fasting hypoglycemia |
| Cushing's syndrome |
| Menstrual disorder |
| High blood level of PAI−1 |
| Hyperleptinemia |
| Neuralgia |

| Disease and Complication |
| --- |
| Type 1 diabetes |
| Type 2 diabetes |
| MODY1 |
| MODY2 |
| MODY3 |
| MODY4 |
| MODY5 |
| Rabson−Mendenhall syndrome |
| Insulin receptor abnormality Type B |
| Insulin receptor abnormality Type A |
| Insulinoma |
| Hyperlow−density lipoproteinemia |
| Persistent hyperinsulinemic hypoglycemia of infancy (PHHI) |

| Clinical Marker Value |
| --- |
| 75g Oral glucose tolerance test (OGTT) 2 hour value (mg/dl) ≧200 |
| Base excess(nmol/l) >−2 |
| beta2 Microglobulin (micro g/day) ≧5000 |
| beta Cartenoid (cartenoid fraction) high value |
| HbA1C(%) ≧6.5 |
| HDLCholesterol(mg/dl)20−35 |
| HLA−DR3(Caucasian) |
| HLA−DR4(Japanese) |
| HLA−DR4(Caucasian) |
| ICA Antibody(+) |
| Increase in Val concentration (plasma) |
| Apolipoprotein BI≧141 |
| Apolipoprotein CII≧7.7 |
| Apolipoprotein CIII≧19.2 |

Example 5

An example of searching a subnet in a molecule-function network from a key molecule involved in diabetes in the pathology-linkage database is shown.

At present, the details of the onset mechanism of diabetes have not been clarified. Therefore, we first search molecules that fluctuate quantitatively and/or qualitatively in diabetes from the pathology-linkage database, and obtain a group of key molecules with descriptions TNF $\alpha$(+), FFA(+), insulin(-), HNF-4 $\alpha$(m), glucose(+), leptin(+), and HbA1C (+). Then, we search subnets to which each key molecule belongs using the biomolecule-information database and biomolecule-linkage database.

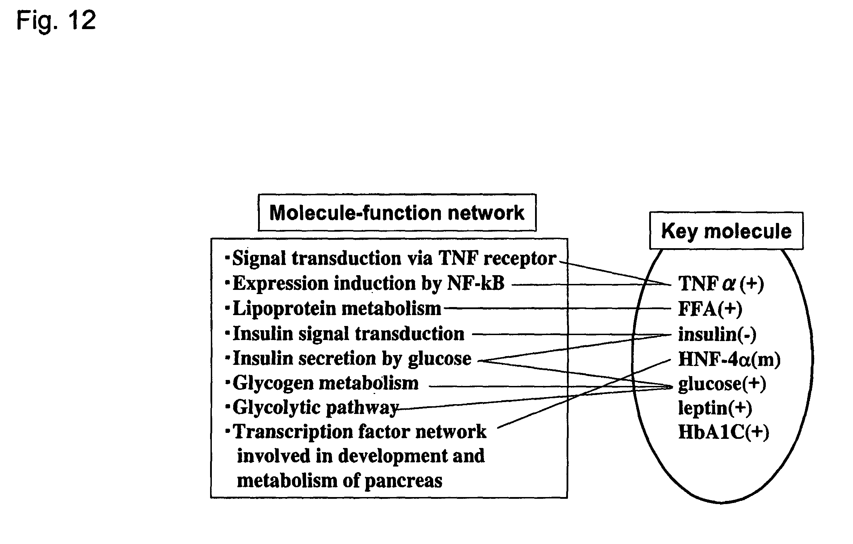

By the above procedures, a group of subnets that might be related to diabetes are obtained for respective key molecules, such as "Signal transduction via TNF receptor" and "Expression induction by NF $\kappa$B" for TNF $\alpha$(+); "Lipoprotein metabolism" for FFA(+); "Insulin signal transduction" and "Insulin secretion by glucose" for insulin(-); "Transcription factor network involved in development and metabolism of pancreas" for HNF-4 $\alpha$(m); "Insulin secretion by glucose", "Glycogen metabolism", and "Glycolytic pathway" for glucose(+) (Fig.12).

With this method, it is possible to examine the relationship between pathways in a life process and molecules related to diabetes or molecules that fluctuate in diabetes.

Example 6

For diabetes, a classification based on onset mechanisms is used at present. The method is based on the progress of recent research of onset mechanisms of diabetes, and is used for the purpose of carrying out more appropriate diagnosis and treatment. Here, an example of analyzing a mechanism of diabetes classified by the onset mechanisms is shown.

Among the diabetes classified as "other special type," MODY1 (maturity onset diabetes of the youth 1) is known. In order to examine how MODY1 is different from other types of diabetes, we search items for MODY1 from the disease and complication group in the pathology-linkage database, carry out a connect search using the pathology-linkage database from the items, and extract related items.

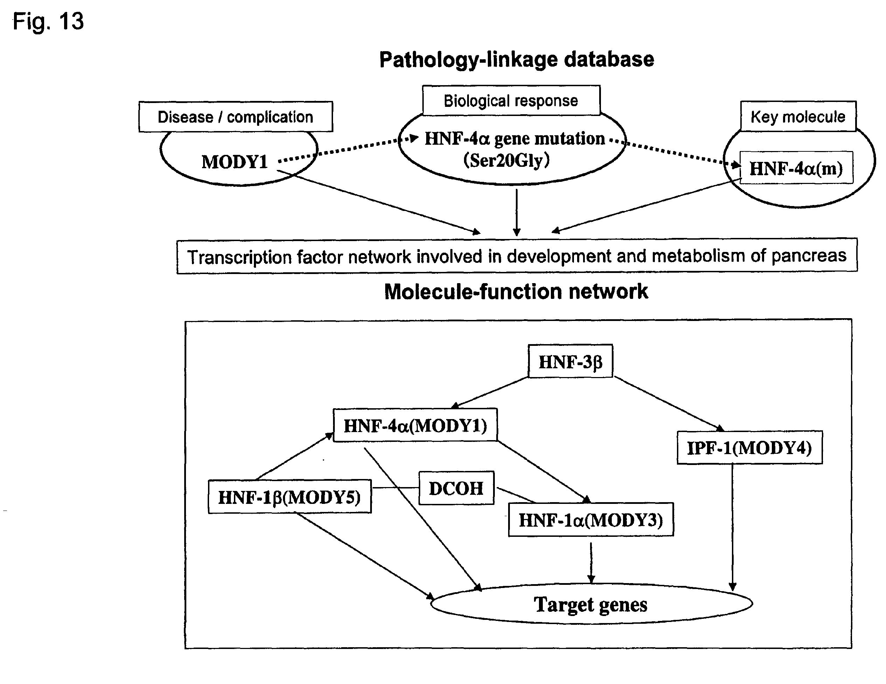

As a result of the above search, HNF-4 $\alpha$(m) corresponding to a causative gene of MODY1 is found in the key molecule group, and "HNF-4 $\alpha$ gene mutation" is found in the biological response group, respectively. Furthermore, using the biomolecule-linkage database, we search a subnet to which the transcription factor HNF-4 $\alpha$ is related, and obtain a subnet "Transcription factor network involved in development and metabolism of pancreas." By generating a molecule-function network belonging to said subnet, it is possible to know the specific biomolecule pairs around HNF-4 $\alpha$ (Fig. 13).

Thus, by using the methods of the present invention, it is possible to obtain information on the molecule and mechanism related to the onset mechanism or information on the difference from other diseases, even from more precisely classified disease names.

Example 7

It is needless to say that diabetes is a disease due to a metabolic disorder; MODY2 is pointed out as an example of the disease where the causal relation between the metabolic system in an organism and diabetes has been directly clarified. Here, we provide an example of a search for the purpose of obtaining sugar metabolism and diabetes, and further its onset mechanism.

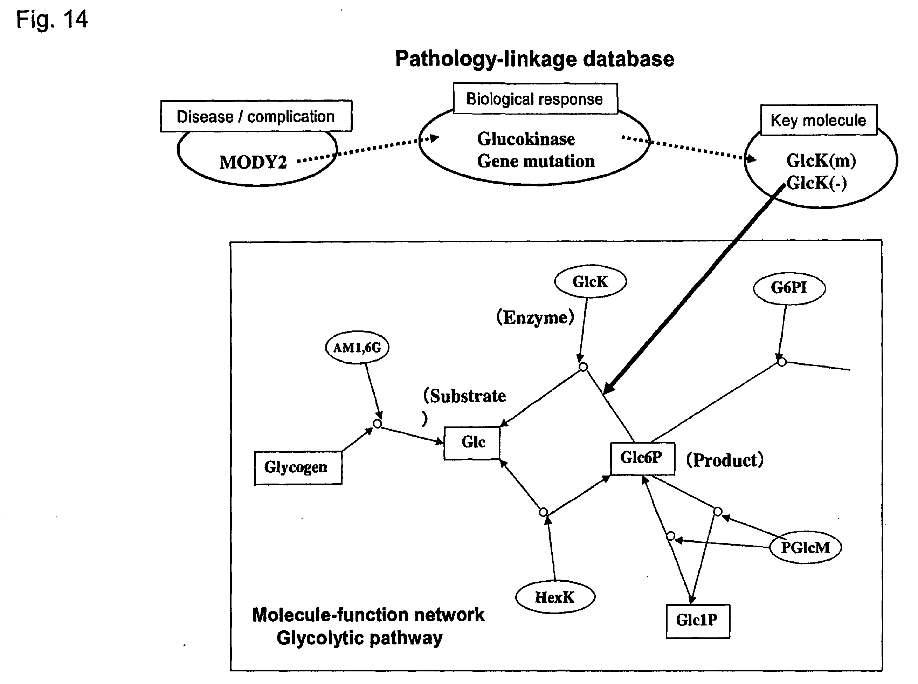

First, we search the pathology-linkage database using MODY2 as a query, and obtain GlcK(m) and GlcK(-) which are the data items of key molecules related to MODY2. Then, we carry out a search with the biomolecule-linkage database using GlcK(m) and GlcK(-) as queries, and extract the subnet "Glycolytic pathway" having the corresponding data to these queries (Fig. 14).

Example 8

In order to examine a role of insulin, which is one of the most important molecules in diabetes, in an organism, we show an example of a search using the databases of the present invention using insulin as a query.

Insulin is related to diabetes in some way. First, we search the pathology-linkage database using insulin as a query, and extract "Insulin(+)", "Insulin(-)" and "Insulin(m)" from the key molecule group; "Decrease of insulin action", "Decrease of insulin secretion potency", and "Decrease of number of insulin receptor" from the biological response group; "Insulin resistance" from the symptom and syndrome group; "Insulin antibody (IAA)(+), insulin injection history (-)" from the clinical marker value group; and "Insulin receptor abnormality type A" from the disease and complication group, respectively.

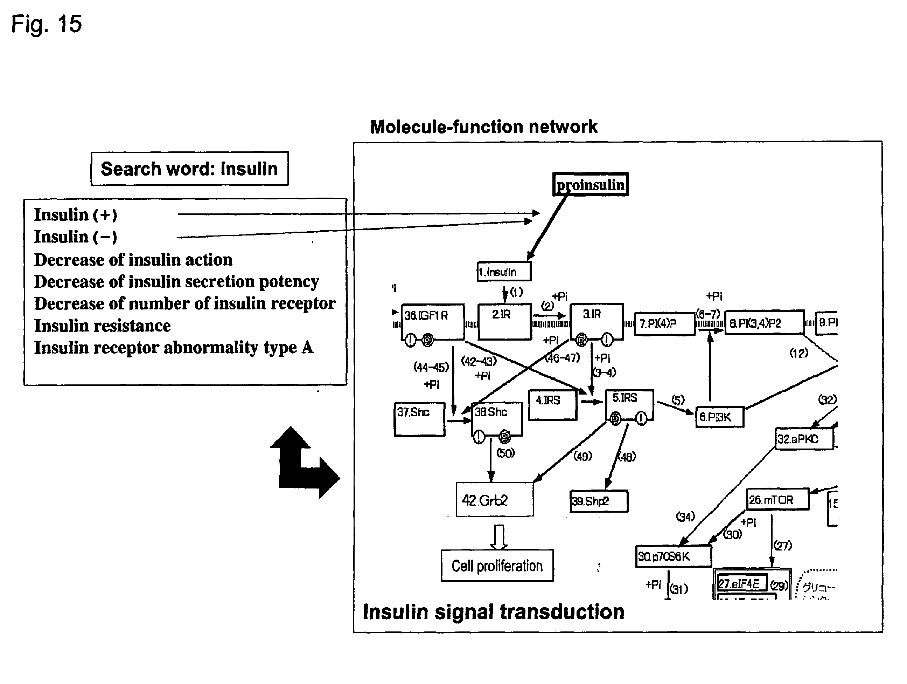

Furthermore, we carry out a search with the biomolecule-linkage database using insulin as a query, and extract "Insulin signal transduction" and "Insulin secretion in the beta cell of pancreas" and others as subnet names. Among them, particularly in the "Insulin signal transduction", it is found that bio-events such as "Cell proliferation", "Protein synthesis", "Glycogen synthesis", "Lipid decomposition", "Glycolytic pathway", "Sugar transport", and "Apoptosis" exist at the downstream of the biomolecule pair of the insulin-insulin receptor (Fig.15). Consequently, it is found that influence of inhibition of this signal transduction by some causes is diverse, and it is possible to know the importance and functional variety of insulin in an organism.

Example 9

In life-style related diseases, there are many cases where several diseases occur simultaneously or contingently and such diseases have common causes. Diabetes per se provokes various complications, however, it is difficult to consider diabetes as a single disease independently in the syndromes so called "deadly quartet" and "syndrome X".

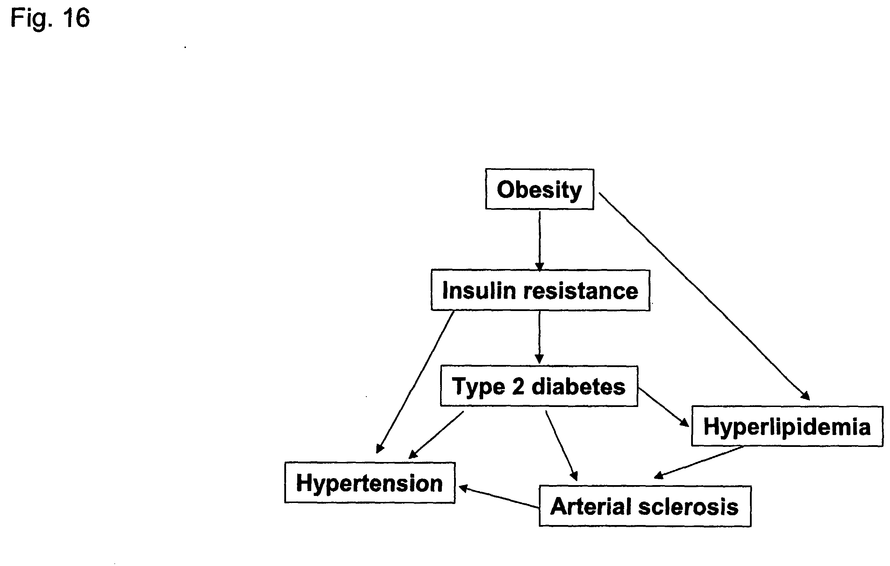

Thus, a connect search of the pathology-linkage database was carried out using a disease condition common to these syndromes, which is "insulin resistance", as a query. By displaying the result of the connect search as a directional graph, it is possible to display diseases to which "insulin resistance" is related and relations among the diseases graphically in an easily understandable way (Fig. 16).

Example 10

An example of finding out a mechanism of action of a certain drug molecule based on the data of the drug molecule information database is shown.

As an example of the information included in the drug molecule information database, data of acarbose whose mechanism of action is sugar absorption retardation and which is used as a diabetic drug is shown in Table 2.

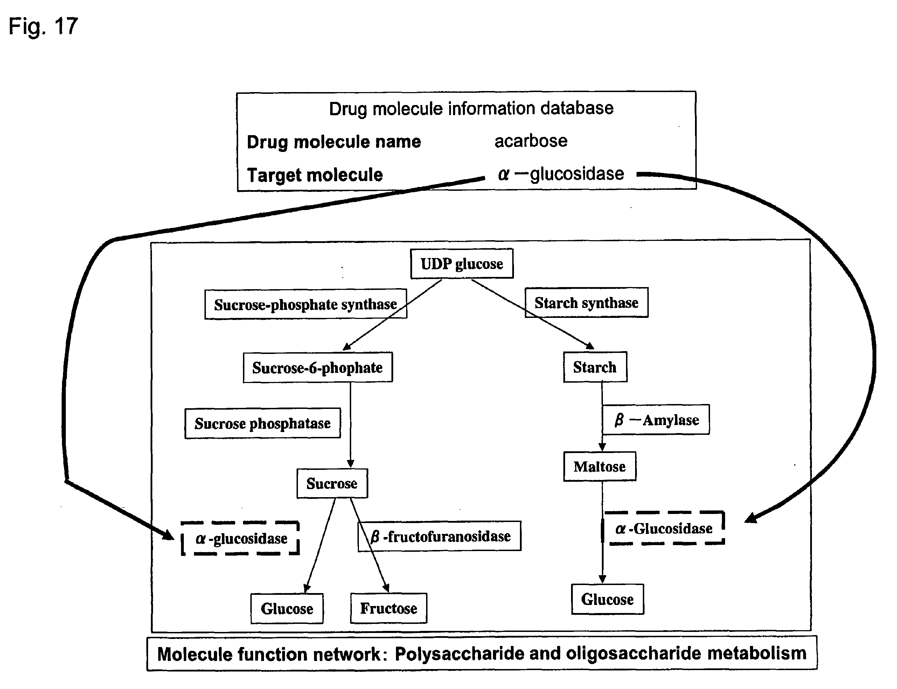

When we try to find out the mechanism of action using acarbose or its commercial name as a query, we can obtain the information that its target biomolecule (target molecule) is $\alpha$-glucosidase by searching the drug molecule information database. Furthermore, by searching the biomolecule-linkage database using $\alpha$-glucosidase as a query, we find out that this molecule is in the subnet called "Polysaccharide and oligosaccharide metabolism". Furthermore, by displaying a molecule network in said subnet, we can understand that $\alpha$-glucosidase catalyzes the catabolic reactions such as those from sucrose to glucose and from maltose to glucose, wherein acarbose inhibits these reactions (Fig.17).

**Table 2**

**Drug Molecule Information Database**

| | |
|---|---|
| Name of drug molecule (Product Name) | Acarbose α-Glucosidase inhibitor |
| Target Molecule | α-Glucosidase |
| Adverse Effect Information | (1) Important adverse effect<br>Seious hepatopathy such as fulminant hepatitis may occur. Serious liver function failure or jaundice involving elevation of AST(GOT) ALT(GPT) may occeur.<br>(2) Other adverse effects<br>edema (particularly female), swelling feeling, increase in LDH, headache, wobble, heavy-headed feeling, abdominal bloating, diarrhea, abdominal pain, gastric pain, gastric heavy feeling, abdominal discomfort (digestive organ), increase in GOT, increase in GPT rise, anemia, leukopenia, thrombocytopenia(blood), rash, hives, itching sensation(hypersensitivity), increase in BUN(kidney) |
| Indication | Type 2 diabetes |
| Comments | Improvement of postprandial hyperglycemia in diabetes (only limited to the cases of when sufficient blood sugar control is not obtained by diet therapy and exercise therapy, or when sufficient blood sugar control is not obtained for patients taking oral hypoglycemic drug or insulin preparation in addition to diet therapy and exercise therapy) |

Example 11

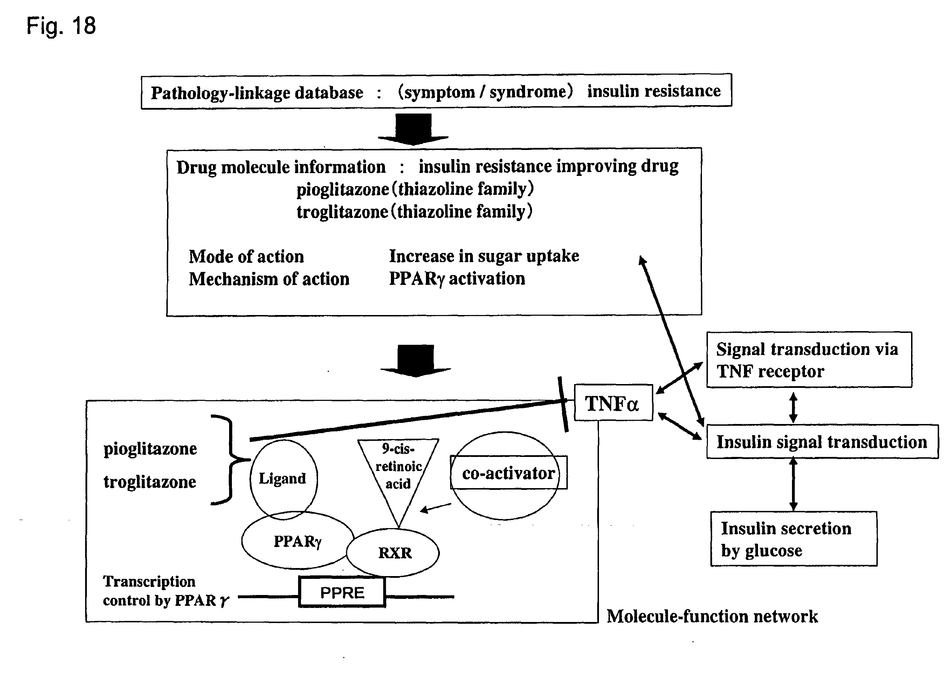

A typical characteristic of type 2 diabetes is insulin resistance. Here, we show an example of a method of examining details of the mechanism of action on a molecule-function network for a drug molecule that is supposed to improve insulin resistance (Fig. 18).

We search in the pathology-linkage database using "insulin resistance" as a query, and find "Insulin resistance" in the symptom and disease group where the focused disease is "Diabetes". Then we search in the drug molecule information database using "insulin resistance" similarly as a query with the condition of partial match of character strings, and extract drug molecules of thiazolidine family, "pioglitazone" and "troglitazone" (withdrawn from the market in Japan now), with pharmacological action of improving insulin resistance. Based on the data in the drug molecule information database, we find out that the mode of action of these insulin resistance improving drugs is "Increase in sugar uptake" and the mechanism of action is "PPARγ (peroxisome proliferator activated receptor γ) activation".

Here, regarding the mode of action of "sugar uptake increase", we further search for a molecule-function network using "sugar uptake" as a query, and find out a bio-event "Sugar uptake" downstream of the molecule GLUT4 in the subnet "Insulin signal transduction."

Industrial Applicability

The biomolecule-linkage database of the present invention which is a collection of information on biomolecule pairs including bio-events is useful for generating a molecule-function network with a necessary range which is a functional or biosynthetic linkage between molecules and predicting bio-events to which an arbitrary biomolecule is related directly or indirectly, and furthermore, by linking it to the information on drug molecules or genetic information, it is possible to obtain necessary knowledge for drug developments and medical treatments based on differences of individuals.

**Claims**

1. A method of generating a molecule-function network comprising a step of a connect search using a database which stores information on biomolecules hierarchized by one or more items including items selected from a group consisting of modification state, active or inactive state, complexation state, and structural change.

2. A method of generating a molecule-function network comprising a step of a connect search using a biomolecule-linkage database wherein information on a biomolecule pair comprises a condition with which the biomolecule pair is formed.

3. A method of generating a molecule-function network comprising a step of a connect search using a pathology-linkage database which stores information on a disease as a grouped and/or hierarchized data item and stores information on correlation between the data items.

4. A method of generating a molecule-function network using a biomolecule-linkage database, comprising a step of a connect search wherein biomolecule pairs are filtered by setting a condition to one or more data items including data items selected from a group consisting of a relation code, a relation-function code, a reliability code, an acting organ and directionality of a biomolecule pair.

5. The method of any one of claims 1 to 4 further comprising a step of scoring the molecule-function network generated by a connect search using a biomolecule-linkage database, based on one or more data items including data items selected from a group consisting of a relation code, a relation-function code, a reliability code, an acting organ and directionality of a biomolecule pair.

6. A method of analyzing a disease-related gene using the method of any one of claims 1 though 5.

7. A method of analyzing a relation between two or more diseases using the method of any one of claims 1 to 5.

8. A method of presuming a mechanism of action and/or a side effect of a drug molecule by preparing a drug molecule information database and/or a drug molecule-biomolecule linkage database using the method of any one of claims 1 to 5.

Fig. 1

Preparation of "biomolecule-linkage database" (including information on biomolecule pairs and bio-events) ⟷ Preparation of "biomolecule information database"

Designate biomolecule or bio-event   Connect search

• Generation of "molecule-function network" to which an arbitrary biomolecule is involved
• Presumption of bio-event

EP 1 492 019 A1

Fig. 2

EP 1 492 019 A1

Fig. 3

EP 1 492 019 A1

Fig. 4

Fig. 5

Preparation of "drug molecule-linkage database" ⟷ Preparation of "drug molecule information database"

Biomolecule-linkage database (including information on biomolecule pairs and bio-events) ⟷ Biomolecule information database

Connect search

Designate biomolecule, drug molecule, or bio-event

Generation of "molecule-function network" in which biomolecule or drug molecule is involved

Correlation with side effect information

Comparison with molecule-function network for other drug molecules

Presumption of interaction between drugs

EP 1 492 019 A1

Fig. 6

Fig. 7

Fig. 8

EP 1 492 019 A1

Fig. 9

EP 1 492 019 A1

Fig. 10

Information on modification varies or is insufficient depending on the information sources

↓

Adjust modification information so that biomolecule pairs are described without contradiction

EP 1 492 019 A1

## Fig. 11

**Biological response**

- Destruction of pancreatic β cell
- Decrease of PI3K activation
- Inhibition of PPARγ expression
- Promotion of apoptosis
- Decrease of insulin receptor activity
- HNF-4 α gene mutation

**Key molecule**

- TNF α (+)
- FFA(+)
- insulin(-)
- HNF-4α(m)
- glucose(+)
- HbA1C(+)

**Diabetes**

**Disease / complication**

- Type 1 diabetes
- Type 2 diabetes
- MODY1
- Diabetic retinopathy

**Symptom / syndrome**

- Obesity
- Insulin resistance
- Glucose toxicity
- Glucose tolerance disorder
- Thirstiness
- Polydipsia

**Clinical marker value**

- ICA Antibody(+)
- Fasting blood sugar (mg/dl)≧126
- Casual blood sugar (mg/dl)≧200
- HbA1C(%)≧6.5

EP 1 492 019 A1

Fig. 12

## Fig. 13

### Pathology-linkage database

Disease / complication

Biological response

Key molecule

MODY1 ······► **HNF-4α gene mutation** ·····► **HNF-4α(m)**
**(Ser20Gly)**

Transcription factor network involved in development and metabolism of pancreas

### Molecule-function network

**HNF-3β**

**HNF-4α(MODY1)**

**IPF-1(MODY4)**

**HNF-1β(MODY5)**     **DCOH**

**HNF-1α(MODY3)**

**Target genes**

EP 1 492 019 A1

## Fig. 14

**Pathology-linkage database**

Molecule-function network
Glycolytic pathway

EP 1 492 019 A1

## Fig. 15

**Molecule-function network**

Search word: insulin

Insulin (+)
Insulin (−)
Decrease of insulin action
Decrease of insulin secretion potency
Decrease of number of insulin receptor
Insulin resistance
Insulin receptor abnormality type A

proinsulin

1.insulin

36.IGF1R   2.IR   3.IR   7.PI(4)P   8.PI(3,4)P2   9.PI

+PI   (2)   +PI   (6-7)   +PI

(44-45)   (42-43)   (46-47)   +PI   (12)
+PI   +PI   (3-4)

37.Shc   38.Shc   4.IRS   5.IRS   6.PI3K   (32)
(5)

(50)   (49)   (48)   32.aPKC

42.Grb2   39.Shp2   26.mTOR
(34)   +PI
(30)   (27)

Cell proliferation   30.p70S6K

+PI   (31)   27.eIF4E   (29)   グリコ—

**Insulin signal transduction**

EP 1 492 019 A1

Fig. 16

## Fig. 17

Drug molecule information database

**Drug molecule name**  acarbose

**Target molecule**  $\alpha$ —glucosidase

UDP glucose

Sucrose-phosphate synthase

Starch synthase

Sucrose-6-phophate

Starch

Sucrose phosphatase

$\beta$ —Amylase

Maltose

Sucrose

$\alpha$ -glucosidase

$\beta$ -fructofuranosidase

$\alpha$ -Glucosidase

Glucose

Fructose

Glucose

Molecule function network: Polysaccharide and oligosaccharide metabolism

EP 1 492 019 A1

Fig. 18

Pathology-linkage database : (symptom / syndrome) insulin resistance

Drug molecule information : insulin resistance improving drug
pioglitazone (thiazoline family)
troglitazone (thiazoline family)

Mode of action          Increase in sugar uptake
Mechanism of action     PPARγ activation

Signal transduction via
TNF receptor

TNFα

pioglitazone
troglitazone

Ligand

9-cis-
retinoic
acid

co-activator

PPARγ     RXR

Insulin signal transduction

Insulin secretion
by glucose

Transcription
control by PPAR γ            PPRE

Molecule-function network

EP 1 492 019 A1

<div align="center">

**INTERNATIONAL SEARCH REPORT**

</div>

| | International application No. |
|---|---|
| | PCT/JP03/02847 |

**A. CLASSIFICATION OF SUBJECT MATTER**
    Int.Cl$^7$  G06F17/30, A61K45/00, A61P43/00

According to International Patent Classification (IPC) or to both national classification and IPC

**B. FIELDS SEARCHED**

Minimum documentation searched (classification system followed by classification symbols)
    Int.Cl$^7$  G06F17/30, A61K45/00, A61P43/00

Documentation searched other than minimum documentation to the extent that such documents are included in the fields searched
    Jitsuyo Shinan Koho          1922-1996   Toroku Jitsuyo Shinan Koho   1994-2003
    Kokai Jitsuyo Shinan Koho    1971-2003   Jitsuyo Shinan Toroku Koho   1996-2003

Electronic data base consulted during the international search (name of data base and, where practicable, search terms used)
    JICST FILE(JOIS), WPI, INSPEC(DIALOG)

**C. DOCUMENTS CONSIDERED TO BE RELEVANT**

| Category* | Citation of document, with indication, where appropriate, of the relevant passages | Relevant to claim No. |
|---|---|---|
| X | HUKAGAWA et al., "Idenshi Seigyo Network Kochiku Shien no tame no Workbench System no Kaihatsu", The Tissue Culture Engineering, Vol.27, No.2, 25 February, 2001 (25.02.01), pages 67 to 71; particularly, table 1, page 70 | 1-8 |
| X | HUKAGAWA, "Idenshi Network Kaiseki no tame no Platform", Dai 18 kai Biotechnology Symposium Yokoshu, 28 September, 2000 (28.09.00), Pages 18 to 21; particularly, 2.3 GUI | 1-8 |

☐ Further documents are listed in the continuation of Box C.      ☐ See patent family annex.

| | |
|---|---|
| * Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br>    22 April, 2003 (22.04.03) | Date of mailing of the international search report<br>    06 May, 2003 (06.05.03) |
| Name and mailing address of the ISA/<br>    Japanese Patent Office | Authorized officer |
| Facsimile No. | Telephone No. |

Form PCT/ISA/210 (second sheet) (July 1998)